(19) Europäisches Patentamt
European Patent Office
Office européen des brevets

(11) EP 1 261 587 B1

# (12) EUROPEAN PATENT SPECIFICATION

(45) Date of publication and mention of the grant of the patent:
**16.07.2003 Bulletin 2003/29**

(21) Application number: **00977556.0**

(22) Date of filing: **22.11.2000**

(51) Int Cl.[7]: **C07D 231/26**, A61K 31/4155, C07D 403/12, A61P 31/18

(86) International application number:
**PCT/EP00/11628**

(87) International publication number:
**WO 01/040194 (07.06.2001 Gazette 2001/23)**

## (54) STILBENE DERIVATIVES, THEIR PREPARATION AND THEIR USE AS ANTIVIRAL AGENTS

STILBEN-DERIVATE, DEREN HERSTELLUNG UND DEREN VERWENDUNG ALS ANTI-VIRALE MITTEL

DERIVES DE STILBENE, PREPARATION ET UTILISATION COMME AGENTS ANTIVIRAUX

(84) Designated Contracting States:
**AT BE CH CY DE DK ES FI FR GB GR IE IT LI LU MC NL PT SE TR**

(30) Priority: **01.12.1999 GB 9928418**

(43) Date of publication of application:
**04.12.2002 Bulletin 2002/49**

(73) Proprietor: **Universität Basel**
**4003 Basel (CH)**

(72) Inventors:
- **KLIMKAIT, Thomas**
  **79539 Lörrach (DE)**
- **HAMY, François**
  **F-68110 Illzach (FR)**

(74) Representative: **Maucher, Wolfgang, Dipl.-Ing.**
**Maucher, Börjes & Kollegen**
**Dreikönigstrasse 13**
**79102 Freiburg (DE)**

(56) References cited:
**EP-A- 0 795 549**
**WO-A-93/16992**
**US-A- 2 221 360**
**US-A- 2 301 333**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 261 587 B1

Printed by Jouve, 75001 PARIS (FR)

## Description

The present invention relates to a novel use or method of use of compounds which are stilbene derivatives, as such or in the form of pharmaceutical compositions, as inhibitors of the interaction of a transcriptional regulator with a retroviral response element or with other viral or cellular structures of nucleic acid or peptidic nature, or as inhibitors of viral infection, which is based on membrane fusion, a use or method of use of said compounds for the treatment of a virus disease, especially a retroviral infection, such as an HIV infection, especially a use or method of use of said compounds as an antiretroviral therapeutic inhibiting the interaction of a transcriptional regulator with a retroviral response element or with other viral or cellular elements, be it nucleic acid or protein, to a process for the manufacture of a pharmaceutical composition comprising said compounds for any one of the uses mentioned above, to a pharmaceutical composition comprising said compounds for any of the uses mentioned above, and/or to said compounds for use in the treatment of the animal (including human) body; the invention also relates to novel compounds of the stilbene derivative type, to processes for the preparation of these compounds and to novel intermediates.

### Background of the invention

Many different retroviruses have been identified and characterized in recent efforts to understand causes for certain diseases. Among these retroviruses are viruses affecting, e.g., cats, such as FIV. monkeys, such as SIV, and humans, such as HTLV-I, or HIV, especially HIV-1 or HIV-2. A characteristic of these retroviruses, as well as many other viruses, is that the transcription of their RNA is controlled by regulatory proteins which bind to hairpin stem-loop RNA conformations present on mRNA's of the retrovirus; in addition Tat protein is recognized as functioning as activator of certain cellular functions independently of the viral response element.

According to WHO estimations, now more than 25 million people are infected with the retrovirus HIV. This infection usually leads to the death of the infected persons due to the development of AIDS and secondary diseases, such as infections, e.g. by Pneumocystis carinii, Candida mycosis, or exacerbating other viral diseases, and/or tumor diseases, such as Kaposi's sarcome.

The virus HIV (especially HIV-1 or HIV-2) is regarded as the causative agent for the complex disease process leading to AIDS. The genome of this virus encodes (inter alia) two regulatory proteins, Tat and Rev, both of which are effective through regulatory pathways that were identified only recently. These regulatory pathways are controlled at the level of protein-RNA interaction, and more recently recognized to also occur at the level of protein/protein or protein/membrane interactions with cellular structures (summarized in: Gallo, R., Proc. Natl. Sci. U.S.A. 96 8324-8326 (1999)).

Two classes of HIV mRNAs can be distinguished. The first of these consists of a doubly spliced. 2 kb mRNA species encodig the viral regulatory proteins, including Tat and Rev. The second class consists of the unspliced (9 kb) and incompletely spliced (4 kb) viral mRNAs that encode the structural proteins of the virion.

Tat induces a marked increase in the steady-state level of viral mRNA. Evidence suggests that this increase can be explained by an increase in the rate of HIV transcription or/and elongation (reflected in the characterisation as "transcription booster"). Newer evidence describes an additional role for Tat in the potent gene activation and membrane expression of cellular coreceptors for the virus, such as e.g. CXCR4, possibly through mechanisms independent of a viral nucleic acid response element.

In the absence of Rev, viral RNA transcripts are fully spliced by the cellular RNA processing machinery prior to export to the cytoplasm. In the presence of Rev, unspliced (Gag) or partially spliced (Env) viral mRNAs evade processing - instead they are exported directly to the cytoplasm. Rev thus induces the efficient export of viral RNA species that are otherwise excluded from the cell cytoplasm.

Both Tat and Rev recognize regulatory elements on the viral mRNA. Tat exercises one of its functions through a sequence termed TAR (for "Trans-Activation Response Region") that comprises a part of the 5'-noncoding region of all HIV mRNAs. This region forms a stable stem-loop structure in vitro. Recent evidence indicates that Tat binds directly to the TAR RNA sequence and that this binding is independent of the nucleotide sequence in the loop, but dependent on the integrity of the upper stem. In addition and through a still uncharacterized mechanism free Tat protein induces the transcription and protein expression of cellular proteins, such as CXCR4 (Huang et al, J. Virol.72, 8952-8960 (1998)). The action of Rev protein is highly sequence specific and requires recognition of an RNA target sequence, the "Rev Responsive Element" (RRE), a highly conserved region in the middle of the viral env gene. The RRE corresponds to a predicted RNA secondary structure of great stability. The viral RNA binding sites of both Tat and Rev map to protein areas which are highly arginine rich (see Calnan, B.J., et al., Science 252, 1167-1171 (1991) and Tiley, L. S., et al., Proc. Natl. Acad. Sci. USA 89, 758-62 (1992)), the cellular activation has not yet been characterized.

The Tat/TAR interaction has been investigated in more detail in the literature (see Calnan, B.J., et al., Science 252, 1167-1171 (1991)) and Calnan, B.J., et al., in: Peptides: Chemistry and Biology, Proc. XII Amer. Peptide Symp., ed. by J.A. Smith and J.E. Rivier, ESCOM Science, Leiden 1992, pp. 685-7). Towards the carboxyl terminus of Tat a highly basic region (residues 48 - 57) is present which appears to be involved in RNA binding. It is of high therapeutic

interest to identify new chemical entities with the ability to bind HIV RNA at sites of specific regulatory elements (TAR and RRE), and compete in this process with the viral Tat and Rev proteins. Disruptions of Tat/TAR and Rev/RRE complexes or blocking additional functions of these small HIV proteins, such as activating cellular HIV-promoting processes, corrupt the regulatory systems essential for viral replication and provide a powerful basis for therapeutic intervention in AIDS patients. Different strains of HIV produce different forms of Tat; however, the the N-terminal amino acid sequence of 72 amino acids is common to all forms. The principle form of Tat (designated as Tat(1-86) herein) consists of ca. 86 amino acids in known linear sequence (see Ratner et al., Nature 313, 277 (1985), which is incorporated by reference herein). Three domains in the protein have been shown to exist by structure/function analysis, including a proline-rich region spanning residues 1-18, a cysteine-rich region spanning residues 22-37, and a basic region of nine amino acid spanning residues 49-57 with the following sequence:
49(L)-Arg-(L)-Lys-(L)-Lys-(L)-Arg-(L)-Arg-(L)-Gln-(L)-Arg-(L)-Arg-(L)-Arg$^{57}$.
This latter basic region is designated as "basic domain" of the HIV Tat protein.

On the other hand, the binding sites for Tat and Rev on the respective mRNA comprise sites with hairpin stem-loop conformations with so-called bulged residues. For example, Tat is introduced to the transcription machinery following direct binding to an RNA stem-loop structure transcribed from the trans-activation responsive region (TAR). Tat recognizes a U-rich bulge sequence located six residues below the apex of the TAR RNA stem-loop.

## Summary of the Invention

A number of new compounds have recently been approved for the treatment of AIDS, especially inhibitors of the retroviral reverse transcriptase and of the retroviral aspartate protease. However, especially in view of the appearence of drug resistance, many of these compounds loose efficiency, and often rather sophisticated administration protocols, e.g. including triple combinations of one aspartate protease inhibitor with two reverse transcriptase inhibitors, are necessary.

It is thus of great advantage to add new compounds of different nature and against different viral targets to the arsenal of substances effective against restroviral diseases, such as AIDS.

US 2,221, 360 (patented Nov. 12, 1940) and US 2,301,333 (patented Nov. 10, 1942) mention stilbene analogues that are useful as dyes and as intermediates for the synthesis of dyes. No pharmaceutical utility is mentioned or suggested.

Surprisingly, it has been found that the compounds mentioned in the two US patents, or analogues thereof, especially the compounds of formula I defined below, show very favourable and valuable pharmaceutical characteristics, especially with regard to the therapeutic and/or diagnostic treatment of retroviral infections, particularly AIDS.

More specifically, the compounds of formula I can be used in the treatment of a retroviral disease in a warm-blooded animal which disease is responsive to the inhibition of the interaction of a transcriptional regulator with a retroviral response element, that is as antiretroviral compounds and/or therapeutics inhibiting the interaction of transcriptional regulators with retroviral response elements, for example the interaction between a transcriptional regulator of HIV with a retroviral response element of HIV, especially the interaction between HIV-1 Tat and TAR and/or the interaction between HIV-1 Rev and RRE. Similarly the compound can be used for .... by inhibiting the interaction of a transcriptional activator, especially HIV Tat with cellular molecules that are directly responsible for expression and function of cellular, e.g. surface molecules such as HIV-coreceptors, especially CXCR4, which are essential for HIV infection.

Due to the mechanism that provides these compounds with an incomparable therapeutic potential to complement or replace existing, specific or less specific antiviral treatments, the use of the compounds of formula I given below is of particularly high value for the treatment of various retroviral infections, e.g. against variants of HIV, especially such variants that have become resistant to other kinds of treatment.

## Detailed description of the invention

A compound to be used according to the invention is preferably a compound of formula I,

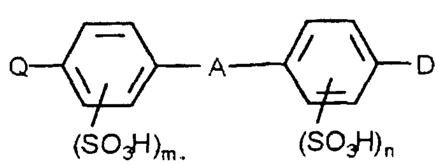

(I)

wherein

A is $-CH_2-CH_2-$ or -CH=CH-;

D is a radical of the partial formula (A),

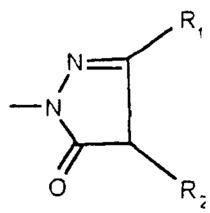

(A)

wherein

$R_1$ is hydrogen, lower alkyl, phenyl or carboxy; and

$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group,

each of **m** and **n** is, independently of the other, 1, 2, 3 or 4; and

**Q** is selected from the group comprising

(a) amino,
(b) nitro,
(c) a radical of the partial formula (B),

-NH-G (B)

wherein

G is selected from the group comprising unsubstituted or oxo substituted lower alkanoyl; benzoyl; phenylaminocarbonyl wherein the phenyl is unsubstituted or substituted by (hydroxy and/or carboxy-substituted phenyl)azo; and naphthylaminocarbonyl wherein naphthyl is unsubstituted or substituted by one or more substituents selected from the group comprising hydroxy, sulfo, phenylazo and phenylazo wherein phenyl is substituted by one or more substituents independently selected from the group comprising hydroxy, carboxy, nitro or sulfo,

(d) a radical of any one of the partial formulae (C), (D) and (E)

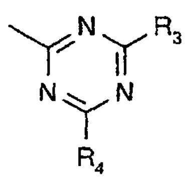

(C),

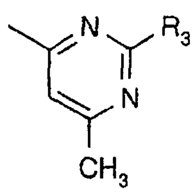

(D),

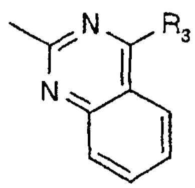

(E)

wherein
$R_3$ is selected from the group comprising

(i) a radical of the partial formula (F)

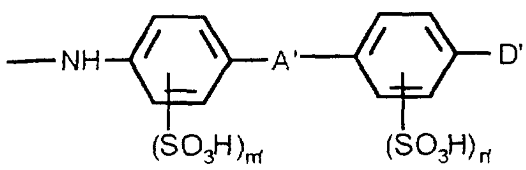

(F)

wherein
A' is $-CH_2-CH_2-$ or -CH=CH-,
D' is hydrogen, sulfo or a radical of the partial formula (A)

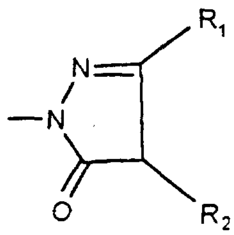

(A)

wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo,
and each of m' and n' is 1, 2, 3 or 4,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,

and
$R_4$ is phenyl, halo, amino, phenylamino, hydroxyphenylamino or (hydroxy and/or sulfo-substituted phenyl)azophenylamino,
(e) a radical of the partial formula (G)

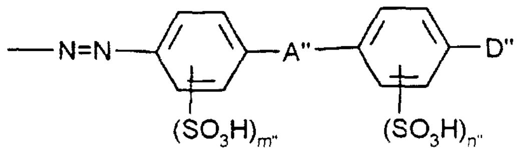

(G)

wherein
A" is $-CH_2-CH_2-$ or -CH=CH-,
D" is a radical of the partial formula (A)

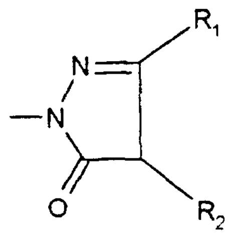

(A)

wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo, and each of m" and n" is 1, 2, 3 or 4,
(f) a radical of the partial formula (H)

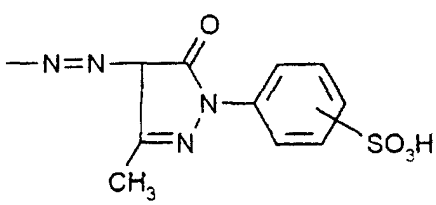

(H);

and
(g) a radical of the partial formula (K),

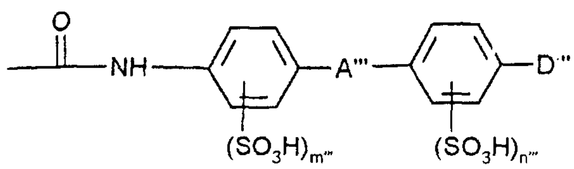

wherein
A''' is -CH2-CH2- or -CH=CH-;
D''' is a radical of the partial formula (A),

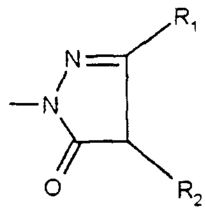

(A)

wherein
$R_1$ is hydrogen, lower alkyl, phenyl or carboxy; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group, and
each of m''' and n''' is 1, 2, 3 or 4;

or a tautomer thereof; or a salt or a complex salt thereof where at least one salt-forming or complex-forming group is present.

Unless otherwise indicated, the general terms and names used in the description of the present invention preferably have the following meanings:

The term "lower" defines a moiety with up to and including maximally 7, especially up to and including maximally 4, carbon atoms, said moiety being branched or straight-chained. Lower alkyl, for example, is methyl, ethyl, n-propyl, sec-propyl, n-butyl, isobutyl, sec-butyl, tert-butyl, n-pentyl, n-hexyl or n-heptyl.

Halo is preferably fluoro, chloro, bromo or iodo, especially chloro.

The compounds of formula I can exist as isomers or mixtures of isomers; for example, if one or more asymmetric carbon atoms are present, these carbon atoms can be in the (R)-, (S)-or (R,S)-configuration, independent of one another; and/or, if one or more double bonds are present in A, A' and/or A", these can be present in the cis/trans, the cis or (preferably) the trans form. It is thus possible to obtain isomeric mixtures, such as racemates or diastereomeric mixtures, or pure diastereomers or enantiomers, depending on the number of asymmetric carbon atoms and on whether isomers or isomeric mixtures are present. Preferred are pure stereoisomers (pure enantiomers, pure diastereomers or pure trans-isomers).

A is preferably -CH=CH-, which is most preferably present in the trans-configuration.

In a radical of the partial formula (A) lower alkyl $R_1$ is preferably methyl and $R_2$ is preferably hydrogen, 5-chloro-2-hydroxy-phenylazo, 2-hydroxy-4-nitro-phenylazo, 2-hydroxy-5-sulfo-phenylazo, 2-carboxy-phenylazo or 2-hydroxy-4-sulfo-1-naphthylazo.

Each of **m** and **n** is preferably 1, the corresponding sulfo group being bonded preferably in the o-position with regard to the bivalent radical -A- in formula I.

If Q is a radical of the partial formula (B) then G is preferably selected from the group consisting of lower alkanoyl, especially acetyl; acetoacetyl; benzoyl; phenylaminocarbonyl; 4-[3-carboxy-4-hydroxy-phenylazo]-phenylaminocarbonyl, 2-(5-hydroxy-7-sulfo-naphthyl)-aminocarbonyl, 2-[5-hydroxy-6-(2-hydroxy-5-sulfo-phenylazo)-7-sulfo-naphthyl]-aminocarbonyl, 2-[5-hydroxy-6-(2-carboxy-phenylazo)-7-sulfo-naphthyl]-aminocarbonyl and 2-[5-hydroxy-6-(2-hydroxy-5-nitro-phenylazo)-7-sulfo-naphthyl]-aminocarbonyl.

In a preferred radical of formula (C), (D) or (E), $R_3$ is preferably selected from

(i) a radical of the partial formula (F)

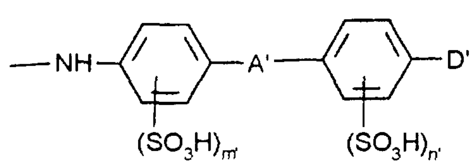

(F)

wherein
A' is -CH=CH-, preferably in the trans form,
D' is hydrogen, sulfo or a radical of the partial formula (A)
wherein

$R_1$ is hydrogen, phenyl, carboxy or preferably methyl; and $R_2$ is hydrogen, 5-chloro-2-hydroxy-phenylazo, 2-hydroxy-4-nitro-phenylazo, 2-hydroxy-5-sulfo-phenylazo, 2-carboxy-phenylazo or 2-hydroxy-4-sulfo-1-naphthylazo,

and each of m' and n' is 1, the corresponding sulfo groups preferably being bonded in o-position with regard to the bivalent radical -A'-;

(ii) phenylamino wherein phenyl is substituted by 4-sulfo-phenylazo, preferably in the p-position, by 3-carboxy-4-hydroxy-phenylazo, preferably in the p-position, by methyl in 3-position and by 3-methyl-4-[(4,8-disulfo)-2-naphthylazo-]-phenylazo in 4-position or by [2-(phenylazo)-2-(acetyl)-acetyl]amino, preferably in the p-position; and

(iii) 1-(3,6-disulfo-naphthyl)amino, 1-(8-hydroxy-3,6-disulfo-naphthyl)amino, 2-(5-hydroxy-7-sulfo-naphthyl)amino, 1-(8-hydroxy-5-(2-hydroxy-5-chloro-phenylazo)-6-sulfo-naphthyl)amino, 1-(7-(2-carboxyphenylazo)-8-hydroxy-3,6-disulfo-naphthyl)-amino, 1-(8-hydroxy-7-(2-hydroxy-4-nitro-phenylazo)-3,6-disulfo-naphthyl)amino or 2-(8-hydroxy-7-(2-hydroxy-5-nitro-phenylazo)-6-sulfo-naphthyl)-amino,

and
$R_4$ is phenyl, chloro, amino, phenylamino, 3-hydroxyphenylamino or 4-(2-hydroxy-4-sulfo-phenylazo)phenylamino.

If Q is a radical of the partial formula (G), then preferably in formula (G)

A" is -CH=CH-,
D" is hydrogen, sulfo or a radical of the partial formula (A) wherein
$R_1$ is hydrogen, phenyl, carboxy or preferably methyl; and
$R_2$ is hydrogen, 5-chloro-2-hydroxy-phenylazo, 2-hydroxy-4-nitro-phenylazo, 2-hydroxy-5-sulfo-phenylazo, 2-carboxy-phenylazo or 2-hydroxy-4-sulfo-1-naphthylazo,
and
each of m" and n" is 1, the corresponding sulfo groups preferably being bonded in o-position with regard to the bivalent radical -A"-.

If Q is a radical of the partial formula (H), then preferably in formula (H) the sulfo group is in p-position.

If Q is a radical of the partial formula (K), then preferably in formula (K)

A"' is -CH=CH-,
D"' is hydrogen, sulfo or a radical of the partial formula (A) wherein
$R_1$ is hydrogen, phenyl, carboxy or preferably methyl; and
$R_2$ is hydrogen, 5-chloro-2-hydroxy-phenylazo, 2-hydroxy-4-nitro-phenylazo, 2-hydroxy-5-sulfo-phenylazo, 2-carboxy-phenylazo or 2-hydroxy-4-sulfo-1-naphthylazo, and
each of m"' and n"' is 1, the corresponding sulfo groups preferably being bonded in o-position with regard to the bivalent radical -A"'-.

The compounds of formula I can exist in the form of tautomers that result from the intramolecular migration of protons. A tautomer of the formula I is especially one wherein any group of the formula (A) is present in the following tautomeric form:

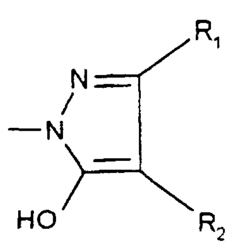

wherein $R_1$ and $R_2$ have the meanings given for a compound of formula I.

In analogy, a group of the formula (H) may be present in the hydroxy form instead of the oxo-form.

These and other tautomeric forms may be present in pure from or as mixtures, depending on the properties of the corresponding compound of formula I, e.g. in the form of equilibrium mixtures. The presence and possible conditions for the existence of these tautomers or further tautomers are known to the person having skill in the art. Any of

these tautomers are comprised by the definition of compounds of formula I.

Salts are preferably pharmaceutically acceptable salts of compounds of formula I.

Such salts are formed preferably from compounds of formula I having an acid group, for example a carboxy group or a sulfo group, and are, for example, salts thereof with suitable bases, such as non-toxic metal salts derived from metals of groups Ia, Ib, IIa and IIb of the Periodic Table of the Elements, especially suitable alkali metal salts, for example lithium, sodium or potassium salts, or alkaline earth metal salts, for example magnesium or calcium salts, also zinc salts or ammonium salts, as well as salts formed with organic amines, such as unsubstituted or hydroxy-substituted mono-, di- or tri-alkylamines, especially mono-, di- or tri-lower alkylamines, or with quaternary ammonium compounds, for example with N-methyl-N-ethylamine, diethylamine, triethylamine, mono-, bis- or tris-(2-hydroxy-lower alkyl)-amines, such as mono-, bis- or tris-(2-hydroxyethyl)amine, 2-hydroxy-tert-butylamine or tris-(hydroxymethyl) methylamine, N,N-di-lower alkyl-N-(hydroxy-lower alkyl)-amines, such as N,N-dimethyl-N-(2-hydroxyethyl)-amine or tri-(2-hydroxyethyl)-amine, N-methyl-D-glucamine, or quaternary ammonium salts, such as tetrabutylammonium salts. The compounds of formula I having a basic group, for example an amino group, can form acid addition salts, for example with inorganic acids, for example hydrohalic acids, such as hydrochloric acid, sulfuric acid or phosphoric acid, or with organic carboxylic, sulfonic, sulfo or phospho acids or N-substituted sulfamic acids, for example acetic acid, propionic acid, glycolic acid, succinic acid, maleic acid, hydroxymaleic acid, methyl maleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, as well as with amino acids, for example the a-amino acids mentioned hereinbefore, especially glutamic acid and aspartic acid, and with methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose-6-phosphate, N-cyclohexylsulfamic acid (forming cycla mates) or with other acidic organic compounds, such as ascorbic acid. A compound of formula I with acid and basic groups can also form internal salts.

For isolation, diagnostic (in vitro) or purification purposes, it is also possible to use pharmaceutically unacceptable salts, for example perchlorates or picrates. Also in the case of a novel compound of fomula I, a pharmaceutically unacceptable salt is encompassed by the present invention.

A complex salt of a compound of formula I is preferably a pharmaceutically acceptable complex salt and may be formed, for example, if a group comprising an o-hydroxyphenyl-azo or o-carboxyphenyl-azo group is present. The possible metal ions include but are not limited to copper, nickel, cobalt, manganese and comparable ions. In the case of a novel compound of fomula I, also a pharmaceutically unacceptable complex salt is encompassed by the present invention.

The term "comprising" preferably can be replaced with the expression "consisting essentially of", more preferably with "consisting of".

Where the term compound of formula I is used herein, this term is intended to mean either the compound as such or, where appropriate and useful, also to include a tautomer thereof, a salt thereof or a complex salt thereof.

The compounds of formula I have useful, in particular pharmacologically useful, properties. Surprisingly, it has been found that the compounds of formula I are able to inhibit the propagation of a retrovirus, especially HIV, especially HIV-1, especially in infected human lymphocytes and show a particularly potent, specific inhibition on the binding of the Tat protein to TAR. In addition compounds of formula I have been found to suppress Tat-mediated activation of cellular co-receptors for HIV. They thus represent a new class of inhibitors and therapeutics.

The in vitro inhibition of the interaction between Tat and TAR can be shown by a competition Tat-TAR gel-shift assay. Through binding of the protein (recombinant Tat, Medical Research Council (MRC), Cambridge, U.K. - for the sequence of recombinant Tat see Churcher et al., J. Mol. Biol. 230, 90-110 (1993)) to the RNA (synthetic TAR duplex, Genset, Paris, France; for the sequence see Hamy et al., J. Mol. Biol. 230, 111-123 (1993)), the overall size and the charge/weight ratio of the formed duplex are changed, so that electrophoretic migration through a native polyacrylamide gel is affected. By radioactive labelling of the RNA and subsequent autoradiography, free RNA and complexes can be discriminated based on their relative positions in the gel (Hamy et al., J. Mol. Biol. 230, 111-123 (1993)). If the binding reaction with a test substance is able to prevent the protein binding to the radiolabelled RNA, this competition for binding can be visualized on the autoradiography as a decreased intensity of the retarded band. In more detail, compounds of the formula I are tested as follows:

The 14-mer strand of duplex TAR-RNA is labelled in the presence of T4 polynucleotide kinase (New England Biolabs, Beverly, MA, USA) using [$\gamma$-$^{32}$P]ATP (Amersham, Little Chalfont, UK), 10 mM DTT (=dithiothreitol), 50 mM Tris.HCl (pH 7.4, Tris = Tris(hydroxymethyl)aminomethane), 0.77 % (w/v) spermidine (Fluka, Switzerland) and 10 units T4 polynucleotide kinase incubated at 37 °C for 20 min. After heat-treatment (65 °C, 10 min) for enzyme inactivation, the unincorporated [$\gamma$-$^{32}$P]ATP is removed by chromatography through a Sephadex NAP-10 column (Pharmacia, Uppsala, Sweden) equilibrated with water. Prior to the in-vitro-binding reaction, the labelled 14-mer is annealed to 1.5 equivalents of unlabelled 17-mer by heating to 90 C for 3 min, followed by slow cooling down to 0 °C. The binding reaction for protein and RNA which takes place in a volume of 25 ml contains approximately 10,000 cpm of the labelled

duplex TAR-RNA and 20 nM recombinant Tat protein in TK buffer (Tris.HCl 20 mM pH 8.0, KCl 50 mM) with 10 mM DTT, 0.1 % Triton X-100 ((Alkylphenylpolyethylenglykol, Rohm & Haas, Darmstadt, Germany) in the absence or presence of varying concentrations of inhibitor. The autoradiographies are quantified by Phosphorimager (Molecular Dynamics/Bucher, Basle, Switzerland). A $CD_{50}$ value is determined as the concentration of a compound of the formula I giving a 50 % decrease in the intensity of the retarded band (Tat-TAR complex). The $CD_{50}$-values that are obtained are preferably in the range of from 10 x $10^{-9}$ to 10 x $10^{-6}$ M, more preferably from 10 x $10^{-9}$ to 0.5 x $10^{-6}$ M.

It is possible to show that similar binding affinity can be found when wild-type unlabelled TAR-RNA is used as competitor, thus suggesting that the compounds of the present invention have affinities comparable to that of the high molecular weight full-length Tat protein in vitro.

The in vitro inhibition of cellular coreceptor expression through HIV Tat can be shown by using reverse transcription of the RNA for the coreceptor, followed by a specific amplification through polymerase chain reaction (RT-PCR). Unstimulated peripheral leukocytes from healthy human donors, not expressing significant amounts of coreceptor RNA, i.e. CXCR4, are incubated with recombinant Tat protein (Huang, L., et al. J. Virol 72, 8952-8960, (1998)). Within five hours, cells upregulate coreceptor expression, whereas untreated cells show no expression. The specific signal for the coreceptor is detected by RT-PCR-amplification of the respective RNA after RNA purification from treated cells (TRIZOL method, Life Technologies, Paisley, UK) Cellular activation with HIV-1 Tat in the presence of said stilbene inhibitor does not result in the production of a specific PCR product. This serves as direct evidence for the suppression of Tat-mediated stimulation of coreceptor-expression by said inhibitor, independently of other viral molecules. Specifically, inhibitory activity of said compound class was measured as reduction of the PCR signal to levels of the control sample, which was not treated with HIV-1 Tat.

The in vitro inhibition of the interaction between Rev and RRE can be shown by a competition Rev-RRE gelshift assay. Through binding of the protein (recombinant Rev, Medical Research Council (MRC), Cambridge, U.K. - for the sequence of recombinant Rev see Heaphy et al., Cell 60, 685-93 (1993)) to the RNA (synthetic RRE duplex, Genset, Paris, France; for the sequence see Iwai et al., Nucl. Acids Res. 20, 6465-72 (1992)), the overall size and the charge/weight ratio of the formed duplex are changed, so that electrophoretic migration through a native polyacrylamide gel is affected. By radioactive labelling of the RNA and subsequent autoradiography, free RNA and complexes can be discriminated based on their relative positions in the gel (Hamy et al., J. Mol. Biol. 230, 111-123 (1993)). If the binding reaction with a substance is able to prevent the protein binding to the radiolabelled RNA, this competition for binding can be visualized on the autoradiography as a decreased intensity of the retarded band. In more detail, compounds of the formula I are tested as follows:

The 14-mer strand of duplex RRE-RNA is labelled in the presence of T4 polynucleotide kinase (New England Biolabs, Beverly, MA, USA) using [γ-$^{32}$P]ATP (Amersham, Little Chalfont, UK), 10 mM DTT (=dithiothreitol), 50 mM Tris.HCl (pH 7.4, Tris = Tris(hydroxymethyl)-aminomethane), 0.77 % (w/v) spermidine (Fluka, Switzerland) and 10 units T4 polynucleotide kinase incubated at 37 °C for 20 min. After heat-treatment (65 °C, 10 min) for enzyme inactivation, the unincorporated [γ-$^{32}$P]ATP is removed by chromatography through a Sephadex NAP-10 column (Pharmacia, Uppsala, Sweden) equilibrated with water. Prior to the in-vitro-binding reaction, the labelled 14-mer is annealed to 1.5 equivalents of unlabelled 15-mer by heating to 90 C for 3 min, followed by slow cooling down to 0 °C. The binding reaction for protein and RNA which takes place in a volune of 25 ml contains approximately 10,000 cpm of the labelled duplex RRE-RNA and 20 nM ecombinant Rev protein in TK buffer (Tris.HCl 20 mM pH 8.0, KCl 50 mM) with 10 mM DTT, 0.1 % Triton X-100 ((Alkylphenylpolyethylenglykol, Rohm & Haas, Darmstadt, Germany) in the absence or presence of varying concentrations of inhibitor. The autoradiographies are quantified by Phosphorimager (Molecular Dynamics/Bucher, Basle, Switzerland). An $IC_{50}$ value is determined as the concentration of a compound of the formula I giving a 50 % decrease in the intensity of the retarded band (Rev-RRE complex). It is possible to show that similar binding affinity can be found when wild-type unlabelled TAR-RNA is used as competitor, thus suggesting that the compounds of the present invention have affinities comparable to that of the high molecular weight full-length Rev protein in vitro.

In addition, the inhibition of viral growth of HIV-1 in cellular systems in vitro can be demonstrated by procedures known in the art, e.g. according to the method described in Lazdins et al., AIDS Research and Human Retroviruses 8 (4), 505-11 (1992). In brief, Peripheral Blood Mononuclear Lymphocytes (PBLs) are obtained from the blood of healthy volunteers by leukapheresis. Cells (1 x $10^6$/ml) are cultured for 2 days in RPMI-1640 (Gibco), supplemented with 10 % heat-inactivated fetal calf serum (Gibco), 50 mg/ml streptomycin, 50 U/ml penicillin (Amimed), 2 nM glutamine and 10 mM hepes buffer (Gibco). Stimulated lymphocytes are obtained by culturing in the presence of PHA (0.25 mg/ml; Wellcome diagnostics, Templehill, Dartford, England). PHA-lymphocyte stimulation is confirmed by the increase in cell size (Scattergram, FACS analysis). Cells are exposed to HIV-1/LAV.04 (see Barre-Sinoussi et al., Science 220, 868-871 (1983)) for 6 h. Following infection, the cells are washed and resuspended in RPMI-1640 (see above) supplemented with human IL-2 (Genzyme, Cambridge, MA) at 0.6 x $10^6$/ml. Medium is changed every 3 days and activity of viral reverse transcriptase (RT activity) is determined in cell supernatants, serving as a measure for the presence of virus and thus of progression of infection according to known procedures (see Willey et al., J. Virol. 62, 353-8 (1991) for

details of the method). In brief, RT determination is possible as follows: The RT activity is determined in 50mM Tris (α, α,α--tris(hydroxymethyl)methylamine, ultra pure, Merck, Federal Republic of Germany) pH 7.8; 75mM of KCl, 2mM of dithiothreitol, 5mM of $MgCl_2$; 0.05% Nonidet P-40 (detergent; Sigma, Switzerland); 50 μg/ml of polyadenylic acid (Pharmacia, Sweden); and 1.6 μg/ml of dT(12-18) (Sigma, Switzerland). The mixture is filtered through an Acrodisc filter (0.45μ: Gellman Science Inc, Ann Arbor) and stored at -20°C. 0.1% (v/v) [α-$^{32}$P]dTTP is added to aliquots of that solution in order to achieve a final radioactive activity of 10μCi/ml. 10μl of the culture supernatant are transferred to a new 96-well microtitre plate and 30μl of the mentioned RT cocktail are added thereto. After mixing, the plate is incubated for from 1.5 to 3hours at 37°C. 5 μl of that reaction mixture are transferred to Whatman DE81 paper (Whatman, Maidstone, UK). The dried filters are washed three times for 5 minutes with 300 mM of NaCl/25mM of trisodium citrate and once with 95% ethanol and again air-dried. Evaluation is effected in a Matrix Packard 96-well counter (Packard, Downers Grove, IL, USA). The RT activity is a measure of the reproduction of HIV-1. The compounds of formula I inhibit virus reproduction when administered in the micromolar range, for example during 17 days after infection practically no increase in RT activity can be determined in the presence of preferably 5 to 50 μM concentrations of an inhibitor of the present invention (for example 0 to 100 counts per minute), while in the control high increase of RT activity can be found (for example more than 9000 counts per minute on day 17). The $ED_{90}$ is defined as that concentration of compound at which 90 % reduction of RT activity versus the untreated control is observed.

Cellular toxicity experiments are conducted with human Peripheral Blood Lymphocytes or macrophages. No cytotoxicity is observed with concentrations up to 100 μM (highest tested concentration of compound of formula I), even in the case of repeated dosing with each change of cultrue medium.

After single application of up to 50 mg/kg by i.v. administration to mice no signs of overt toxicity are observed during 8 days of observation. In detail, the assay is conducted as follows: The assay is designed for the determination of the maximal tolerated dose (MTD) For the MTD determination, three six to eight week old female Balb/c AnCbif Tif mice (Ciba animal farm, Sisseln, Switzerland) per dose group are treated once i.v. The dosage is increased until toxic effects are observed within 7 days after drug treatment. After 7 days the animals are euthanasized and organs are macroscopically inspected. The MTD is the dose where all three animals still survive. For example, when the compounds of examples 2 or 4, trans-5-(Acetoacetylamino)-2-[2-{4-(4,5-dihydro-3-methyl-5-oxo-1H-pyrazol-1-yl)-2-sulfophenyl]ethenyl]-benzenesulfonic acid or N,N'-Bis(trans-4-{2-[4-(3-methyl-5-oxo-4,5-dihydro-1H.-pyrazol-1-yl)-2-sulfo-phenyl]-vinyl}-2-sulfophenyl)-[1,3,5]triazine-2,4,6-triamine, are dissolved in sterile sodium chloride solution 0.9%, the MTD after i.v. administration is > 50 mg/kg.

It can be shown that inhibition by a compound of formula I is mainly due to its ability to permeate into cells, a fact that can be demonstrated by a Fusion Induced Gene Stimulation Assay (FIGS-assay) that can also assess cell surface effects and be used to determine the underlying mechanism(s) of action.

The compounds of formula I can thus, for example, be used in the treatment of retroviral diseases in warm-blooded animals which diseases are responsive to the inhibition of the interaction of transcriptional regulators with retroviral response elements, preferably HIV-, such as HIV-1-, infections which are responsive to the inhibition of the interaction between Tat and TAR and/or Rev and RRE or by blocking additional functions of these small HIV proteins, such as activating cellular HIV-promoting processes, especially of activation of cellular coreceptors for HIV; and more specifically for the treatment of AIDS and its initial stages, such as ARDS, in humans by inhibition of the interaction between Tat and TAR and/or Rev and RRE of HIV-1.

Also treatment of infected cells, e.g. lymphocytes, outside the body is possible in order to reintroduce healthy cells by transplantantion or injection, for example in order to improve the lymphocyte titer in patients with advanced AIDS.

The compounds of formula I can also be used in the treatment of commercially valuable cell (such as lymphocyte) cultures against retroviral infections, especially against HIV, such as HIV-1, infections. This is of special advantage where, for example, special antibodies, vaccines or signaling molecules, such as interleukines or the like, are produced from such cells which are, therefore, of large commercial value.

Cells from infected cells can be treated in analogy to the method described above for Peripheral Blood Mononuclear Lymphocytes (PBLs).

In general, the present invention relates also to the use of a compound of formula I in the inhibition of the interaction of transcriptional regulators with retroviral response elements, especially as mentioned above, or to a method wherein a compound of formula I is used to inhibit the interaction of transcriptional regulators with retroviral response elements. The invention also relates to the use of a compound of formula I in the inhibition of cellular pathways directly responsive to viral transactivators, i.e. HIV Tat, especially cellular coreceptors, which are essential for HIV infection, such as CXCR4 (Alkhatib, G, et al. Science 272, 1955 (1996); Feng, Y., et al. Science 272, 872 (1996)).

The compounds of the invention may be used either on their own or in combination - either as fixed combinations or by separate administration of the single active ingredients or combinations thereof in combination, e.g. in a chronically staggered manner, with other pharmacologically active substances, for example together with inhibitors of reverse transcriptase (especially nucleoside analogues, especially 3'-azido-3'-deoxypyrimidine (= Zidovudine = ®RETROVIR, Burroughs-Wellcome), 2',3'-dideoxycytidine (= Zalcitabine = ®HIVID, Hoffmann-LaRoche), 2',3'-dideoxyinosine (= Didanosine = ®VIDEX, Bristol-Myers-Squibb) or (2R,cis)-4-amino-1- (2-hydroxymethyl-1,3-oxathiolan-5-yl) -(1H)-pyrimidin-2-one (= Lamivudine, Glaxo), or non-nucleoside analogues, such as 11-cyclopropyl-5,11-dihydro-4-methyl-(6H)-dipyrido [3,2-b;2',3'-e]-[1,4]diazepin-6-one); and/or inhibitors of retroviral aspartate proteases (especially (a) an inhibitor mentioned in EP 0 346 847 (published on Dec. 20, 1989) and EP 0 432 695 (published on June 19, 1991); corresponds to US 5,196,438), especially Ro 31-8959 (= Saquinavir; Hoffmann-LaRoche); (b) an inhibitor mentioned in EP 0 541 168 (published on May 12, 1993; corresponds to US 5,413,999), especially L-735,524 (= Indinavir = ®CRIXIVAN; Merck & Co., Inc.); (c) an inhibitor mentioned in EP 0 486 948 (published on May 27, 1992; corresponds to US 5,354,866), especially ABT-538 (= Ritonavir; Abbott)); (d) a compound named KVX-478 (or VX-478 or 141W94; GlaxoWellcome, Vertex und Kissei Pharmaceuticals); (e) a compound named AG-1343 (Agouron); (f) a compound named KNI-272 (Nippon Mining); (f) a compound named U-96988 (Upjohn); and/or a compound named BILA-2011 BS (= Palinavir; Boehringer-Ingelheim); or in each case a salt thereof where a salt forming group is present.

In the case of the preferred subjects of the invention mentioned hereinafter, instead of the general expressions there may be used the more specific definitions mentioned at the beginning, where appropriate and expedient.

The invention preferably relates to the use of a compound of formula I, a tautomer thereof, a (preferably pharmaceutically acceptable) salt or a (preferably pharmaceutically acceptable) complex salt thereof as described hereinbefore anc hereinafter as an inhibitor of the interaction of a transcriptional regulator with a retroviral response element, more preferably as an inhibitor of the interaction between a transcriptional regulator of HIV with a retroviral response element of HIV, especially as an inhibitor for the interaction between HIV-1 Tat and TAR and/or the interaction between HIV-1 Rev and RRE; especially in cells outside the body of a warm-blooded animal including a human, but also in a warm-blooded animal, including a human; or to the use of a compound of formula I, a tautomer thereof, a (preferably pharmaceutically acceptable) salt or a (preferably pharmaceutically acceptable) complex salt thereof as decribed hereinbefore and hereinafter for the manufacture of a pharmaceutical preparation for the inhibition of the interaction of a transcriptional regulator with a retroviral response element, more preferably as an inhibitor of the interaction between a transcriptional regulator of HIV with a retroviral response element of HIV, especially as an inhibitor for the interaction between HIV-1 Tat and TAR or by blocking additional functions of these small HIV proteins, such as activating cellular HIV-promoting processes, especially of cellular coreceptors for HIV and/or the interaction between HIV-1 Rev and RRE; especially in cells outside the body of a warm-blooded animal including a human, but also in a warm-blooded animal, including a human.

The invention preferably also relates to a method of inhibiting the interaction of a transcriptional regulator with a retroviral response element, more preferably the interaction between a transcriptional regulator of HIV with a retroviral response element of HIV, especially the interaction between HIV-1 Tat and TAR or inhibiting additional specific functions of Tat, such as activating cellular HIV-promoting processes, especially expression of cellular coreceptors for HIV, especially CXCR4, and/or the interaction between HIV-1 Rev and RRE, in a human being, wherein a compound of formula I, a tautomer thereof, a (preferably pharmaceutically acceptable) salt or a (prefereably pharmaceutically acceptable) complex salt thereof as described hereinbefore and hereinafter is administered especially to a warm-blooded animal, including a human, in need of such treatment or to a cell outside the body of a warm-blooded animal , especially outside the human body, in need of such treatment, in an amount that is sufficient for inhibiting said interaction. In the case of inhibition in cells outside a warm-blooded animal, administtration to cells is e.g. under routine conditions.

The invention preferably also relates to the use of a compound of formula I, a tautomer thereof, a (preferably pharmaceutically acceptable) salt or a (preferably pharmaceutically acceptable) complex salt thereof as described hereinbefore and hereinafter for the treatment of a virus disease, especially a retroviral infection, such as an HIV infection, most especially an HIV-1 infection, especially in cells outside the body of a warm-blooded animal including a human, but also in a warm-blooded animal, including a human; or to the use of a compound of formula I, a tautomer thereof, a (preferably pharmaceutically acceptable) salt or a (preferably pharmaceutically acceptable) complex salt thereof as decribed hereinbefore and hereinafter for the manufacture of a pharmaceutical preparation for the treatment of a virus disease, especially a retroviral infection, such as an HIV infection, most especially an HIV-1 infection, especially in cells outside the body of a warm-blooded animal including a human, but also in a warm-blooded animal, including a human.

The invention preferably also relates to a method of treating a virus disease, especially a retroviral infection, such as an HIV infection, most especially an HIV-1 infection, especially in cells outside the body of a warm-blooded animal including a human, but also in a warm-blooded animal, including a human, wherein a compound of formula I, a tautomer thereof, a (preferably pharmaceutically acceptable) salt or a (prefereably pharmaceutically acceptable) complex salt thereof as described hereinbefore and hereinafter is administered especially to a warm-blooded animal, including a human, that is in need of such treatment, or to a cell outside the body of a warm-blooded animal, especially outside the human body, that is in need of such treatment, in an amount that is sufficient for the treatment of said disease.

More particularly, the invention relates to the use of a compound of formula I, a tautomer thereof, a (preferably pharmaceutically acceptable) salt or a (preferably pharmaceutically acceptable) complex salt thereof as described

hereinbefore and hereinafter as an antiretroviral therapeutic (also for prophylaxis with regard e.g. to uninfected cells) inhibiting the interaction of a transcriptional regulator with a retroviral response element, especially as a therapeutic against HIV-1 inhibiting the interaction of the Tat/TAR interaction or inhibiting additional functions of Tat, such as activating cellular HIV-promoting processes, especially of cellular coreceptors for HIV, especially CXCR4, especially in cells outside the body of a warm-blooded animal including a human, but also in a warm-blooded animal, including a human; or to the use of a compound of formula I, a tautomer thereof, a (preferably pharmaceutically acceptable) salt or a (preferably pharmaceutically acceptable) complex salt thereof as decribed hereinbefore and hereinafter for the manufacture of a pharmaceutical preparation useful as an antiretroviral therapeutic (also for prophylaxis with regard e.g. to uninfected cells) inhibiting the interaction of a transcriptional regulator with a retroviral response element, especially as a therapeutic against HIV-1 inhibiting the interaction of the Tat/TAR interaction or inhibiting additional specific functions of Tat, such as activating cellular HIV-promoting processes, especially expression of cellular coreceptors for HIV, especially CXCR4, especially in cells outside the body of a warm-blooded animal including a human, but also in a warm-blooded animal, including a human.

More particularly, the invention also relates to a method of use of a compound of formula I, a tautomer thereof, a (preferably pharmaceutically acceptable) salt or a (preferably pharmaceutically acceptable) complex salt thereof as described hereinbefore and hereinafter, as an antiretroviral therapeutic inhibiting the interaction of a transcriptional regulator with a retroviral response element, especially as a therapeutic against HIV-1 inhibiting the interaction of the Tat/TAR interaction or inhibiting additional specific functions of Tat, such as activating cellular HIV-promoting processes, especially expression of cellular coreceptors for HIV, especially CXCR4,, comprising administering said compound of formula I, a tautomer thereof, a (preferably pharmaceutically acceptable) salt or a (preferably pharmaceutically acceptable) complex salt thereof as described hereinbefore and hereinafter, especially to a cell outside the body of a warm-blooded animal, including a human, that is in need thereof, in an amount that is effective in said inhibition but also in a warm-blooded animal, including a human, that is in need thereof, in an amount that is effective in said inhibihtion.

The invention preferably also relates to a process for the manufacture of a pharmaceutical composition comprising a compound of formula I, a tautomer thereof, a pharmaceutically acceptable salt or a pharmaceutically acceptable complex salt thereof as described hereinbefore and hereinafter and a pharmaceutically acceptable carrier, for use as an inhibitor of the interaction of a transcriptional regulator with a retroviral response element, more preferably as an inhibitor of the interaction between a transcriptional regulator of HIV with a retroviral response element of HIV, especially as an inhibitor for the interaction between HIV-1 Tat and TAR or for additional specific functions of Tat, such as activating cellular HIV-promoting processes, especially expression of cellular coreceptors for HIV, especially CXCR4, and/or the interaction between HIV-1 Rev and RRE; for use in the treatment of a virus disease, especially a retroviral infection, such as an HIV infection, most especially an HIV-1 infection; or as an antiretroviral therapeutic (also for prophylaxis with regard e.g. to uninfected cells) inhibiting the interaction of a transcriptional regulator with a retroviral response element, especially as a therapeutic against HIV-1 inhibiting the interaction of the Tat/TAR interaction or additional specific functions of Tat, such as activating cellular HIV-promoting processes, especially expression of cellular coreceptors for HIV, especially CXCR4,; in a warm-blooded animal, especially a human, or in a cell outside the body of a warm-blooded animal, including a human; said process comprising admixing a compound of the formula i and at least a pharmaceutically acceptable carrier.

The invention preferably also relates to a pharmaceutical preparation, comprising a compound of formula I, a tautomer thereof, a pharmaceutically acceptable salt or a pharmaceutically acceptable complex salt thereof as described hereinbefore and hereinafter and a pharmaceutically acceptable carrier, for use as an inhibitor of the interaction of a transcriptional regulator with a retroviral response element, more preferably as an inhibitor of the interaction between a transcriptional regulator of HIV with a retroviral response element of HIV, especially as an inhibitor for the interaction between HIV-1 Tat and TAR or of additional specific functions of Tat, such as activating cellular HIV-promoting processes, especially expression of cellular coreceptors for HIV, especially CXCR4, and/or the interaction between HIV-1 Rev and RRE; for use in the treatment of a virus disease, especially a retroviral infection, such as an HIV infection, most especially an HIV-1 infection; or as an antiretroviral therapeutic (also for prophylaxis with regard e.g. to uninfected cells) inhibiting the interaction of a transcriptional regulator with a retroviral response element, especially as a therapeutic against HIV-1 inhibiting the interaction of the Tat/TAR interaction or inhibiting additional specific functions of Tat, such as activating cellular HIV-promoting processes, especially expression of cellular coreceptors for HIV, especially CXCR4,; in a warm-blooded animal, especially a human, or in a cell outside the body of a warm-blooded animal, including a human.

The invention prefereably also relates to a compound of formula I, a tautomer thereof, a pharmaceutically acceptable salt or a pharmaceutically acceptable complex salt thereof as described hereinbefore and hereinafter and a pharmaceutically acceptable carrier, for use in the treatment of the animal or human body, especially for use as an inhibitor of the interaction of a transcriptional regulator with a retroviral response element, more preferably as an inhibitor of the interaction between a transcriptional regulator of HIV with a retroviral response element of HIV, especially as an inhibitor for the interaction between HIV-1 Tat and TAR or for additional specific functions of Tat, such as activating

cellular HIV-promoting processes, especially expression of cellular coreceptors for HIV, especially CXCR4, and/or the interaction between HIV-1 Rev and RRE; for use in the treatment of a virus disease, especially a retroviral infection, such as an HIV infection, most especially an HIV-1 infection; or as an antiretroviral therapeutic (also for prophylaxis with regard e.g. to uninfected cells) inhibiting the interaction of a transcriptional regulator with a retroviral response element, especially as a therapeutic against HIV-1 inhibiting the interaction of the Tat/TAR interaction or inhibiting additional specific functions of Tat, such as activating cellular HIV-promoting processes, especially expression of cellular coreceptors for HIV, especially CXCR4; in a warm-blooded animal, especially a human, or in a cell outside the body of a warm-blooded animal, including a human.

Preferably, the compound to be used is a compound of formula I wherein

**A** is -CH=CH-, preferably in the trans configuration;
**D** is a radical of the partial formula (A),
wherein
$R_1$ is hydrogen, lower alkyl, especially methyl, phenyl or carboxy, preferably methyl;
and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitrophenylazo, 2-hydroxy-5-sulfo-phenylazo, 2-hydroxy-4-sulfo-1-naphthylazo and especially 2-carboxy-phenylazo;
each of **m** and **n** is 1, the corresponding sulfo group being preferably bonded in the o-position with regard to the bivalent radical -A- in formula I; and
**Q** is selected from the group consisting of

(i) amino,
(ii) a radical of the partial formula (B), wherein G is selected from the group comprising unsubstituted or oxo substituted lower alkanoyl, especially acetoacetyl; a radical of the partial formula (C)
wherein
$R_3$ is a radical of the partial formula (F)
wherein
A' is -CH=CH-, preferably in the trans-configuration,
D' is hydrogen, sulfo or preferably a radical of the partial formula (A)
wherein
$R_1$ is hydrogen, lower alkyl, especially methyl, phenyl or carboxy, preferably methyl; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitro-phenylazo, 2-hydroxy-5-sulfo-phenylazo, 2-hydroxy-4-sulfo-1-naphthylazo and especially 2-carboxy-phenylazo;
and each of m' and n' is 1, the corresponding sulfo group being preferably bonded in the o-position with regard to the bivalent radical -A- in formula I; and
$R_4$ is halo, especially chloro, or amino,
and
(iii) a radical of the partial formula (K)
wherein
A''' is -CH=CH-, preferably in the trans-configuration,
D''' is hydrogen, sulfo or a radical of the partial formula (A) wherein
$R_1$ is hydrogen, phenyl, carboxy or preferably methyl; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitro-phenylazo, 2-hydroxy-5-sulfo-phenylazo, 2-hydroxy-4-sulfo-1-naphthylazo and especially 2-carboxy-phenylazo;
and each of m''' and n''' is 1, the corresponding sulfo group being preferably bonded in the o-position with regard to the bivalent radical -A'''- in formula I,

or a tautomer thereof; or a (prefereably pharmaceutically acceptable) salt or a (preferably pharmaceutically acceptable) complex salt thereof where at least one salt-forming or complex-forming group is present.

Especially preferred is the use of a compound mentioned below in the Examples, or a tautomer thereof; or a pharmaceutically acceptable salt or a pharmaceutically acceptable complex salt thereof where at least one salt-forming or complex-forming group is present.

The invention relates also to novel compounds of formula I and their salts and to the use thereof in the processes, methods and pharmaceutical compositions mentioned hereinbefore and hereinafter, and to pharmaceutical compositions comprising those compounds, especially the compounds mentioned below:

Preferred among these compounds according to the invention is a compound of formula I, wherein

**A** is $-CH_2-CH_2-$ or -CH=CH-;

**D** is a radical of the partial formula (A),
wherein
$R_1$ is hydrogen, lower alkyl, phenyl or carboxy; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group,
each of **m** and **n** is 1, 2, 3 or 4; and

**Q** is a radical of of the partial formulae (C),
wherein
$R_3$ is selected from the group comprising

(i) a radical of the partial formula (F),
wherein
A' is $-CH_2-CH_2-$ or -CH=CH-,
D' is hydrogen, sulfo or a radical of the partial formula (A)
wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo,
and each of m' and n' is 1, 2, 3 or 4, preferably 1,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,
and
$R_4$ is amino,

or a tautomer thereof; or a salt or a complex salt thereof where at least one salt-forming or complex-forming group is present.

More preferred is a compound of the formula I wherein

A is -CH=CH-, especially in the trans configuration;
D is a radical of the partial formula (A) wherein
$R_1$ is lower alkyl, especially methyl, and
$R_2$ is hydrogen,
each of **m** and **n** is 1, the corresponding sulfo group being preferably bonded in the o-position with regard to the bivalent radical -A- in formula I; and
Q is a radical of the partial formula (C),
wherein
$R_3$ is a radical of the partial formula (F),
wherein
A' is -CH=CH-, especially in the trans configuration,

D' is a radical of the partial formula (A)
wherein
R1 is lower alkyl, especially methyl; and
R2 is hydrogen,
each of **m** and **n** is 1, the corresponding sulfo group being preferably bonded in the o-position with regard to the bivalent radical -A- in formula I;
and
$R_4$ is amino,
or a tautomer thereof; or a salt or a complex salt thereof where at least one salt-forming or complex-forming group is present.

Especially preferred is the compond of the formula I named N,N'-bis(4-{2-[4-(3-methyl-5-oxo-4,5-dihydro-1.H.-pyrazol-1-yl)-2-sulfo-phenyl]-vinyl}-2-sulfophenyl)-[1,3,5]triazine-2,4,6-triamine (especially in the trans form) or a tautomer thereof; or a pharmaceutically acceptable salt or a pharmaceutically acceptable complex salt thereof where at least one salt-forming or complex-forming group is present.

A compound of the formula I, a tautomer thereof, or a pharmaceutically acceptable salt or complex salt thereof may be prepared in analogy to or by a process described int the literature (see any one of US 2,221,360 (patented Nov. 12, 1940) and US 2,301,333 (patented Nov. 10, 1942), both of which are herewith included for reference). Also starting materials or conversions of a compound of formula I into a different compound of formula I can be found in former publications (see US 2,221,360 or US 2,301,333), or they are commercially available, known or can be prepared according to known procedures.

The new compounds of formula I can be prepared in accordance with methods that, in principle, are known per se in the sense of an analogy process wherein a compound of the formula II,

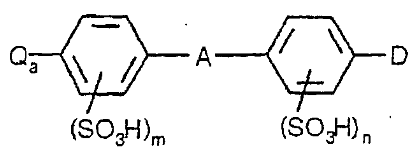

(II)

wherein
$Q_a$ is a radical of the partial formula (C),

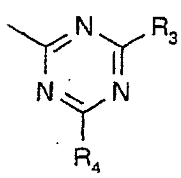

(C),

wherein
$R_3$ is selected from the group comprising

(i) a radical of the partial formula (F),
wherein
A' is $-CH_2-CH_2-$ or -CH=CH-,
D' is hydrogen, sulfo or a radical of the partial formula (A)
wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo,
and each of m' and n' is 1, 2, 3 or 4, preferably 1,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,

and

$R_4$ is halo, especially chloro, and

and A, D, m and n have the meanings given above for new compounds of formula I; (especially a compound of formula II wherein

**A** is $-CH_2-CH_2-$ or -CH=CH-;

**D** is a radical of the partial formula (A),

wherein

$R_1$ is hydrogen, lower alkyl, phenyl or carboxy; and

$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group,

each of **m** and **n** is 1, 2, 3 or 4; and

**Q** is a radical of of the partial formulae (C),

wherein

$R_3$ is selected from the group comprising

(i) a radical of the partial formula (F),

wherein

A' is $-CH_2-CH_2-$ or -CH=CH-,

D' is hydrogen, sulfo or a radical of the partial formula (A)

wherein

R1 is hydrogen, lower alkyl, phenyl or carboxy; and

R2 is hydrogen, phenylazo or naphthylazo wherein the phenyl or napthtyl is unsubstituted or substituted by one or more substituents selected from the group comprising halo, hydroxy, carboxy, nitro and sulfo,

and each of m' and n' is 1, 2, 3 or 4, preferably 1,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,

and

$R_4$ is halo, especially chloro,)

or a tautomer thereof; or a salt or a complex salt thereof where at least one salt-forming or complex-forming group is present,

is reacted with ammonia,

and, if desired, a free compound of formula I or a tautomer thereof obtainable according to this process is converted into a salt or a complex salt, an obtainable salt or a complex salt of a compound of formula I or a tautomer thereof is converted into a different salt or complex salt or into a free compound of formula I or a tautomer thereof, and/or a mixture of isomers of an obtained compound of formula I is separated into individual isomers.

The reaction preferably takes place in appropriate solvents, especially water in the absence of presence of further polar solutes, such as alcohols, e.g. methanol, ethanol or isopropanol, in the presence of preferably 2 to 50 percent by weight ammonia, for example in water with 20 to 35 % ammonia, preferably in a closed reaction vessel, especially a closed tube, at elevated temperatures, preferably in the range from 25 to 100 °C, especially from 60 to 90 °C.

A starting material of the formula II is either known or can be prepared according to methods known in the art (see especially US 2,221,360 (patented Nov. 12, 1940) and US 2,301,333 (patented Nov. 10, 1942)).

Most preferred is a process that s identical or analogous to the processes described in the examples.

The conversion of a free compound of formula I into a salt or a complex salt; or of a salt or a complex salt of a compound of formula I into a different salt or a different complex salt is possible, for example, in solvents, especially in organic solvents, more especially in polar organic solvents, very especially in esters, for example lower alkanoyl-lower alkyl esters, such as ethyl acetate, in amides, for example N,N-di-lower alkyl-lower alkanoylamides, such as dimethylformamide, in alcohols, for example hydroxy-lower alkanes, such as methanol, ethanol, ethylene glycol or glycerol, or aryl alcohols, such as phenols, for example phenol, or in dimethyl sulfoxide, in the absence or presence of water, or in water. Special preference is given to reaction in alcohols, such as the last-mentioned hydroxy-lower alkanes, in mixtures of such alcohols and water, or in water itself.

The reaction is carried out, for example, in free solution, but it may also be effected over chromatographic columns, for example by gel filtration.

The reaction is carried out at temperatures from immediately above the freezing point to the boiling point of the solutions in question, preferably at from 0 to 50°C, especially at from 20 to 40°C, for example at room temperature, in the presence or absence of a protecting gas, such as nitrogen or argon.

The compounds of formula I and the salt-forming salt are used in suitable molar ratios, or the salt forming salt is employed in excess. Preferably, the individual components are used in the molar ratio that corresponds to the ratio of the molarity of the compound of formula I and the counterion(s) in the resulting salts.

The salts that are formed precipitate, for example, by themselves, in some cases only after cooling, or they are precipitated by the addition of solvents, especially of non-polar solvents, for example ethers, such as diethyl ether, or of water, and/or are obtained by partial or complete concentration by evaporation.

The reaction may also be effected via a free compound of formula I, which is prepared, for example, by converting the salt or complex salt of a compound of formula I with (a) first counterion(s) into the free compound with the aid of an acid or base, for example a hydroxy base, such as an alkali metal hydroxide, for example NaOH or KOH, or with an $OH^-$-charged ion exchanger in aqueous solution in the presence or absence of an organic solvent, as defined above; the subsequent conversion of the free compound may be carried out, for example, as described above.

The free compounds of formula I are preferably prepared as just described, also by chromatography, for example by gel filtration, or over ion exchangers.

Mixtures of isomers obtainable according to the invention can be separated in a manner known per se into the individual isomers; diastereoisomers or cis/trans-isomers can be separated, for example, by partitioning between polyphasic solvent mixtures, recrystallisation and/or chromatographic separation, for example over silica gel, and racemates can be separated, for example, by the formation of salts with optically pure salt-forming reagents and separation of the mixture of diastereoisomers so obtainable, for example by means of fractional crystallisation, or by chromatography over optically active column materials.

Pharmaceutical compositions and the preparation thereof

With regard to pharmaceutical compositions that comprise (preferably a novel) compound of formula I, a tautomer thereof; or a pharmaceutically acceptable salt or complex salt thereof, as active ingredient, special preference is given to compositions for enteral, such as nasal, buccal, rectal or, especially, oral, and parenteral, such as intravenous, intramuscular or subcutaneous, administration to warm-blooded animals, especially humans. The compositions comprise the active ingredient on its own or, preferably, together with a pharmaceutically acceptable carrier. The dose of active ingredient depends on the disease to be treated, and on the species, its age, weight and individual condition, on individual pharmacokinetic conditions, and on the mode of administration.

The invention relates also to processes for, and to the use of (preferably the novel) compounds of formula I in the preparation of pharmaceutical compositions that comprise compounds of formula I as active component (active ingredient).

Preferred as active ingredient is always a novel compound of formula I, as defined above.

The pharmacologically acceptable compounds of the present invention may be used, for example, for the preparation of pharmaceutical compositions that comprise an effective amount of the active ingredient together or in admixture with a significant amount of inorganic or organic, solid or liquid, pharmaceutically acceptable carriers.

The pharmaceutical compositions comprise from approximately 1% to approximately 95% active ingredient, dosage forms that are in single dose form preferably comprising from approximately 20% to approximately 90% active ingredient, and dosage forms that are not in single dose form preferably comprising from approximately 5% to approximately 20% active ingredient. Unit dose forms are, for example, dragées, tablets, ampoules, vials, suppositories or capsules. Other dosage forms are, for example, ointments, creams, pastes, foams, tinctures, lipsticks, drops, sprays, dispersions, etc.. Examples are capsules comprising from approximately 0.05g to approximately 1.0g of the active ingredient.

The pharmaceutical compositions of the present invention are prepared in a manner known per se, for example

by means of conventional mixing, granulating, confectioning, dissolving or lyophilising processes.

There are preferably used solutions of the active ingredient, additionally also suspensions or dispersions, especially isotonic aqueous solutions, dispersions or suspensions, which, for example in the case of lyophitised compositions comprising the active ingredient on its own or together with a carrier, e.g. mannitol, may be prepared before use. The pharmaceutical compositions may be sterilised and/or may comprise excipients, for example preservatives, stabilisers, wetting agents and/or emulsifiers, solubilisers, salts for regulating the osmotic pressure and/or buffers, and are prepared in a manner known per se, for example by means of conventional dissolving or lyophilising processes. The said solutions or suspensions may comprise viscosityincreasing substances, such as sodium carboxymethylcellulose, carboxymethylcellulose, dextran, polyvinylpyrrolidone or gelatin, or also solubilisers, for example ®Tween 80 [polyoxyethylene(20) sorbitan monooleate; trademark of ICI Americas, Inc, USA].

Suspensions in oil comprise as the oil component the vegetable, synthetic or semisynthetic oils customarily used for injection purposes. There may be mentioned especially liquid fatty acid esters which contain as acid component a long-chain fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid, where appropriate with the addition of antioxidants, for example vitamin E, b-carotene or 3,5-di-tert-butyl-4-hydroxytoluene. The alcohol component of those fatty acid esters has not more than 6 carbon atoms and is a mono- or poly-valent, for example mono-, di- or tri-valent, alcohol, for example methanol, ethanol, propanol, butanol or pentanol or their isomers, but especially glycol and glycerol. Accordingly, there may be mentioned as examples of fatty acid esters: ethyl oleate, isopropyl myristate, isopropyl palmitate, "Labrafil M2375" (polyoxyethylene glycerol trioleate from Gattefossé, Paris), "Labrafil M1944 CS" (unsaturated polyglycolised glycerides prepared by alcoholysis of apricot kernel oil and composed of glycerides and polyethylene glycol esters; Gattefossé, France), "Labrasol" (saturated polyglycolised glycerides prepared by alcoholysis of TCM and composed of glycerides and polyethylene glycol esters; Gattefossé, France) and/or "Miglyol812" (triglyceride of saturated fatty acids having a chain length of from $C_8$ to $C_{12}$ from Hüls AG, Germany), but especially vegetable oils, such as cottonseed oil, almond oil, olive oil, castor oil, sesame oil, soybean oil and, more especially, groundnut oil.

The preparation of the injection compositions is carried out in customary manner under sterile conditions, as well as the introduction, for example, into ampoules or vials and the sealing of the containers.

Pharmaceutical compositions for oral administration can be obtained, for example, by combining the active ingredient with one or more solid carriers, granulating a resulting mixture, where appropriate, and processing the mixture or granules, if desired, where appropriate with the addition of additional excipients, to form tablets or dragée cores.

Suitable carriers are especially fillers, such as sugars, for example lactose, saccharose, mannitol or sorbitol, cellulose preparations and/or calcium phosphates, for example tri calcium phosphate or calcium hydrogen phosphate, and also binders, such as starches, for example corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches and also carboxymethyl starch, cross-linked polyvinylpyrrolidone, or alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Dragée cores can be provided with suitable, where appropriate enteric coatings, there being used inter alia concentrated sugar solutions, which may comprise gum arabic, talc, polyvinylpyrrolidone, polyethylene glycol and/or titanium dioxide, or coating solutions in suitable organic solvents or solvent mixtures or, for the preparation of enteric coatings, solutions of suitable cellulose preparations, such as acetylcellulose phthalate or hydroxypropylmethylcellulose phthalate. Colourings or pigments may be added to the tablets or dragée coatings, for example for identification purposes or to indicate different doses of active ingredient.

Pharmaceutical compositions for oral administration are also hard gelatin capsules, and soft sealed capsules consisting of gelatin and a plasticiser, such as glycerol or sorbitol. The hard gelatin capsules may comprise the active ingredient in the form of granules, for example in admixture with fillers, such as corn starch, binders and/or glidants, such as talc or magnesium stearate, and, where appropriate, stabilisers. In soft capsules the active ingredient is preferably dissolved or suspended in suitable liquid excipients, such as fatty oils, paraffin oil or liquid polyethylene glycols or fatty acid esters of ethylene glycol or propylene glycol, it likewise being possible to add stabilisers and detergents, for example of the polyoxyethylene sorbitan fatty acid ester type.

Other oral dosage forms are, for example, syrups prepared in customary manner which comprise the active ingredient, for example, in suspended form and in a concentration of approximately from 5% to 20%, preferably approximately 10% or in a similar concentration that provides a suitable single dose when administered, for example, in a measure of 5 or 10ml. Also suitable are, for example, powdered or liquid concentrates for the preparation of shakes, for example in milk. Such concentrates may also be packed in single dose quantities.

Suitable rectally administrable pharmaceutical compositions are, for example, supposi tories that consist of

a combination of the active ingredient with a suppository base. Suitable suppository bases are, for example, natural or synthetic triglycerides, paraffin hydrocarbons, polyethylene glycols or higher alkanols.

For parenteral administration there are suitable, especially, aqueous solutions of an active ingredient in water-soluble form, for example in the form of a water-soluble salt, or aqueous injection suspensions that comprise viscosity-increasing substances, for example sodium carboxymethylcellulose, sorbitol and/or dextran, and, if desired, stabilisers. The active ingredient, where appropriate together with excipients, can also be in the form of a lyophilisate and be made into a solution prior to parenteral administration by the addition of suitable solvents.

Solutions used, for example, for parenteral administration can also be used as infusion solutions.

Preferred preservatives are, for example, antioxidants, such as ascorbic acid, or micro bicides, such as sorbic acid or benzoic acid.

Ointments are oil-in-water emulsions that comprise up to 70%, but preferably from 20 to 50%, water or aqueous phase. There are suitable as the fatty phase especially hydro carbons, for example paraffin oil or hard paraffins, which, in order to improve the water-binding capacity, preferably contain suitable hydroxy compounds, such as fatty alcohols or esters thereof, for example cetyl alcohol or wool wax alcohols, such as wool wax. Emulsifiers are corresponding lipophilic substances, such as sorbitan fatty acid esters, for example sorbitan oleate and/or sorbitan isostearate. Additives to the aqueous phase are, for example, humectants, such as polyalcohols, for example glycerol, propylene glycol, sorbitol and/or polyethylene glycol, or preservatives and perfumes.

Fatty ointments are anhydrous and comprise as base especially hydrocarbons, for example paraffin or paraffin oil, also natural or partially synthetic fats, for example coconut fatty acid triglyceride, or preferably hardened oils, for example hydrogenated groundnut oil or castor oil, also fatty acid partial esters of glycerol, for example glycerol mono- and/or di-stearate, and also, for example, the fatty alcohols increasing water absorption, emulsifiers and/or additives mentioned in connection with the ointments.

Creams are oil-in-water emulsions that comprise more than 50% water. As oily base there are used especially fatty alcohols, for example lauryl, cetyl or stearyl alcohol, fatty acids, for example palmitic or stearic acid, liquid to solid waxes, for example isopropyl myristate, wool wax or beeswax, and/or hydrocarbons, for example Vaseline® (petrolatum) or paraffin oil. Suitable emulsifiers are surface-active substances having predominantly hydrophilic properties, such as non-ionic emulsifiers, for example fatty acid esters of polyalcohols or ethylene oxide adducts thereof, such as polyglyceric acid fatty acid esters or polyethylene sorbitan fatty acid esters, and also polyoxyethylene fatty alcohol ethers or fatty acid esters, or corresponding ionic emulsifiers, such as alkali metal salts of fatty alcohol sulfates, for example sodium lauryl sulfate, sodium cetyl sulfate or sodium stearyl sulfate, which are usually used in the presence of fatty alcohols, for example cetyl alcohol or stearyl alcohol. Additives to the aqueous phase are inter alia agents that reduce the drying out of the creams, for example polyalcohols, such as glycerol, sorbitol, propylene glycol and/or polyethylene glycols, also preservatives and perfumes.

Pastes are creams and ointments having secretion-absorbing powder constituents, such as metal oxides, for example titanium oxide or zinc oxide, also talc and/or aluminium silicates, the purpose of which is to bind any moisture or secretions present.

Foams are administered from pressurised containers and are liquid oil-in-water emulsions in aerosol form, there being used as propellants halogenated hydrocarbons, such as chloro-fluoro-lower alkanes, for example dichlorodifluoromethane and dichlorotetrafluoroethane, or preferably non-halogenated gaseous hydrocarbons, air, $N_2O$ or carbon dioxide. As oil phase there are used inter alia the oil phases used above under ointments and creams, likewise the additives mentioned therein.

Tinctures and solutions generally have an aqueous-ethanolic base to which there are added inter alia polyalcohols, for example glycerol, glycols and/or polyethylene glycol, as humectants for reducing evaporation, and fat-restoring substances, such as fatty acid esters with low molecular weight polyethylene glycols, that is to say lipophilic substances that are soluble in the aqueous mixture, as a replacement for the fatty substances removed from the skin by the ethanol, and, if necessary, other excipients and additives.

In the above-mentioned methods and use processes, the compounds of formula I may be administered prophylactically or therapeutically as such or in the form of pharmaceutical compositions, preferably in an amount that is effective against the mentioned diseases, to a warm-blooded animal, for example a human, requiring such treatment, the compounds being used especially in the form of pharmaceutical compositions.

The pharmaceutical compositions according to the invention are those for enteral, such as nasal, rectal or oral, or parenteral, such as intramuscular or intravenous, administration to warm-blooded animals (humans and animals), that comprise an effective dose of the pharmacological active ingredient, alone or together with a significant amount of a pharmaceutically acceptable carrier. The dose of the active ingredient depends on the species of warm-blooded animal, the body weight, the age and the individual condition, individual pharmacokinetic data, the disease to be treated and the mode of administration.

The dose to be administered to warm-blooded animals, for example humans of approximately 70 kg body weight, is from approximately 3 mg to approximately 3 g, preferably from approximately 10 mg to approximately 1.5

g, for example approximately from 100 mg to 1000 mg per person per day, divided preferably into 1 to 3 single doses which may, for example, be of the same size. Usually, children receive half of the adult dose.

In the case of a methods of treatment of cells outside the body of a warm-blooded animal, a compound of formula I, a tautomer thereof, a salt thereof or a complex salt thereof is prefereably administered in a concentration in the range from 100 nM to 100 μM, preferably from 1 to 50 μM, in the respective culture medium.

Examples

Embodiments of the invention are described in the following specific examples which are not to be construed to be intended to limit the scope of the invention in any way, but serve merely for illustration:

Temperatures, if not mentioned: room temperature or ambient temperature. In mixtures, relations of parts of solvent or eluent or reagent mixtures in liquid form are given as volume relations (v/v), if not indicated otherwise.

Special abbreviations used are:

| | |
|---|---|
| DMSO | dimethyl sulfoxide |
| $d_6$-DMSO | perdeuterated dimethyl sulfoxide |

Measurement of HPLC retention time:

Gradient 95 % A/5% B → 100% B within 15 min;

Reversed phase Nucleosil $C_{18}$ column (Macherey & Nagel, Düren, Germany), 5 μm average bead diameter, 10 nm average pore diameter; column dimensions 4.6 x 250 mm;
flow rate 1 ml/min; detection at 254 nm;
A = 0.1 % trifluoroacetic acid in water, B = 0.1 % trifluoroacetic acid in acetonitrile.

Mass spectroscopy: Measurement on VG-Plattform II from Fisons; electron spray.

$^1$H-NMR: Results are obtained on a Varian 300.

The designation of the compounds in the examples as trans-compounds is tentative - it cannot be excluded that cis-siomers are present.

Example 1: 5-Amino-2-[2-{4-(4,5-dihydro-3-methyl-5-oxo-1H-pyrazol-1-yl)-2-sulfophenyl]-ethenyl]-benzenesulfonic acid (trans)

50 ml of water, 100 g of iron chips and 4 g of acetic acid are boiled under reflux for 15 min. During 1 h, the diazosulfonic acid solution obtained according to 1 a) is added at 90 °C. The reaction solution is stirred at 90 °C for 1 h; then, 7 g of sodium carbonate are added, and the reaction solution is filtered. The hot filtrate is poured into a mixture of 100 g of ice and 170 g of concentrated sulphuric acid. The reaction mixture is stirred at 101-103 °C for 1 h, cooled down to 60 °C, and the precipitating hydrazine sulfonic acid intermediate is filtered off and washed twice with 200 ml of water. The hydrazine sulfonic acid intermediate so obtained is then dissolved in 150 ml of water and 58 g of 30 % aqueous sodium hydroxide. 30 g of acetoacetic acid ethyl ester are added, the reaction mixture is stirred for 30 min, then 14 g of sodium carbonate are added. The reaction solution was then stirred at 95 °C during 2 h. After that, 70 g of 30 % aqueous hydrogen chloride are added and the reaction mixture is cooled overnight. The precipitated product is then filtered off, washed with 100 ml of water and dried at 70 to 80 °C, giving the title compound: HPLC retention time 6.0 min; MS [M-H] = 450; $^1$H-NMR ($d_6$-DMSO) δ = 8.2 ppm (d, 1H, CH-stilbene); 8-7.6 ppm (m, 6H, aryl); 7.2 ppm (d, 1H, CH-stilbene); 5.35 ppm (s, 1H, CH-pyrazolyl); 2.1 ppm (s, 3H, $CH_3$).

1 a) 5-Diazo-2-[2-{4-(4,5-dihydro-3-methyl-5-oxo-1H-pyrazol-1-yl)-2-sulfophenyl]ethenyl]-benzenesulfonic acid solution (trans)

80 g of trans-4-amino-4'-nitro-stilbene-2,2-disulfonic acid (prepared by partial reduction of trans-4,4'-dinitrostilbene-2,2'-disulfonic acid by means of sodium sulfide, see ) are dissolved in 300 ml of water with 27 g of 30 % sodium hydroxide at 50 °C. The solution is cooled down to 0 °C, and then 56 g of 25 % sodium nitrite solution are added. The resulting reaction solution is stirred overnight. After adding 75 g of ice, the reaction mixture is poured on 200 g of ice in 115 g of 30 % hydrochloric acid during 5 min. The resulting mixture is stirred for 1 h at 0 to 5 °C. The reaction mixture is then poured slowly into a solution resulting from the combination of a solution of 70 g of sodium carbonate in 50 ml of water and 80 g of 40% aqueous sodium hydrogen sulfite. After allowing the solution to stand overnight, 30 g of sulphuric acid diluted to 40 % with ice are added. The resulting diazosulfonic acid solution is used

directly in the next reaction step.

Example 2: trans-5-(Acetoacetylamino)-2-[2-{4-(4,5-dihydro-3-methyl-5-oxo-1H-pyrazol-1-yl)-2-sulfophenyl}ethenyl]-benzenesulfonic acid

10 mg of the title compound of Example 1 are dissolved in 500 µl of DMSO, 5 µl of ethyl acetoacetate (Aldrich, Buchs, Switzerland) and 10 µl of triethylamine are added. The reaction mixture is stired at 50 °C for 2h and then worked up by concentration under reduced pressure and subsequent addition of 500 ml petrol ether/diethylether 2:1 (v/v), yielding the title compound as a precipitate: HPLC retention time 7 min; $^1$H-NMR ($d_6$-DMSO/$D_2O$ = 4:1): δ = 2.45 ppm (s, 3H, $CH_3$-acetoacetyl); δ= 2.2 ppm (s, 3H, $CH_3$ pyrazolyl ring).

Example 3: N,N'-Bis(trans-4-{2-[4-(3-methyl-5-oxo-4,5-dihydro-1.H.-pyrazol-1-yl)-2-sulfophenyl]-vinyl}-2-sulfophenyl)-2-chloro-[1,3,5]triazine-4,6-diamine

500 mg of the title compound of Example 1 dissolved in 10 ml of water with 116 mg of sodium carbonate are slowly added to a fine suspension of cyanuric chloride (Aldrich, Buchs, Switzerland) in 5 ml of water at 5 °C. The resulting reaction mixture is then stirred at 60 °C. During the reaction, it is ensured that the reaction mixture remains just neutral by addition of 10 % sodium carbonate solution in the amounts required for this purpose. After no more sodium carbonate addition is necessary to keep the solution neutral, the reaction mixture is cooled down to 0 °C, and 30 % aqueous hydrogen chloride solution is added carefully until the product precipitates. The precipitate is filtered off, washed with 2 ml of cold water and dried at 60 °C, yielding the title compound: HPLC retention time 6.3 min; $^1$H-NMR ($d_6$-DMSO/$D_2O$ = 1:1): δ = 8.15 ppm (d, 2H, CH-stilbene); 7.6-8.1 ppm (m, 12 H, aryl); 7.35 ppm (d, 2H, CH-stilbene); 2.15 ppm (s, 6H, $CH_3$); MS: ($M^{2-}$) = 507.

Example 4: N,N'-Bis(trans-4-{2-[4-(3-methyl-5-oxo-4,5-dihydro-1.H.-pyrazol-1-yl)-2-sulfophenyl]-vinyl}-2-sulfophenyl)-[1,3,5]triazine-2,4,6-triamine

20 mg of the title compound of Example 3 are dissolved in 2 ml of 25 % $NH_3$, and the mixture is heated to 80 °C and kept at that temperature for 8 h in a closed tube. Most of the ammonia in excess is then removed by steam distillation. The product is precipitated by adding 10 % hydrochloric acid, filtered off and dried at 80 °C under reduced pressure, yielding the title compound: HPLC retention time 6.4 min; $^1$H-NMR ($d_6$-DMSO/$D_2O$ = 1:1): δ = 8.1 ppm (d, 2H, CH-stilbene); 7.6-8.05 ppm (m, 12 H, aryl); 7.3 ppm (d, 2H, CH-stilbene); 2.1 ppm (2, 6H, $CH_3$); MS: ($M^{2-}$) = 497.

Example 5: N,N'-Bis(trans-4-{2-[4-(3-methyl-5-oxo-4-(o-carboxyphenylazo)-4,5-dihydro-1.H.-pyrazol-1-yl)-2-sulfophenyl]-vinyl}-2-sulfophenyl)-urea tetrasodium salt

The title compound is synthesized in accordance with a procedure disclosed in the literature (see US 2,221,360 of Nov. 12, 1990, Example 1), starting from 2-amino benzoic acid (Fluka, Buchs, Switzerland) and the title compound of Example 1.
Physical data of the title compound: HPLC retention time 10.5 min; $^1$H-NMR ($d_6$-DMSO/$D_2O$ = 1:1): δ = 8.35 ppm (d, 2H, CH-stilbene); 7.5-8.1 ppm (m, 20 H, aryl); 7.2 ppm (t, 2H, CH-stilbene); 2.3 ppm (s, 6H, $CH_3$).

Example 6: N,N'-Bis(trans-4-{2-[4-(3-methyl-5-oxo-4-(o-carboxyphenylazo)-4,5-dihydro-1.H.-pyrazol-1-yl)-2-sulfophenyl]-vinyl}-2-sulfophenyl)-urea copper complex

100 mg of the title compound of Example 5 are dissolved in 100 ml of water at 90 °C. 20 g of sodium acetate are added. During 1 h, a 20 % copper sulfate solution is added continuously until precipitation of the title compound copper salt is observed. After addition of sodium chloride as such, the precipitated product is filtered off, washed with 1 ml of cold water and dried at 70 °C under vacuum, yielding the title compound: HPLC retention time 10.4 min.

Example 7: In vitro inhibition of the interaction between Tat and TAR:

In accordance with the method described above in detail for the Tat-TAR gel-shift assay, the following $CD_{50}$ values are obtained for the compounds of the Examples given above:

| Compound of Example | $CD_{50}$ (nM) |
|---|---|
| 2 | 20 |

(continued)

| Compound of Example | $CD_{50}$ (nM) |
|---|---|
| 3 | 50 |
| 4 | 50 |
| 5 | 70 |
| 6 | 200 |

Example 8: Inhibition of viral growth of HIV-1 in cellular systems:

According to the method described in detail above for the determination of HIV-1 growth in human Peripheral Blood Mononuclear Lymphocytes (PBLs), the following $ED_{90}$ values are obtained for the compounds of the Examples given above:

| Compound of Example | $ED_{90}$ (µM) |
|---|---|
| 2 | 30 |
| 3 | 20 |

Example 9: Gelatine solution:

A sterile-filtered aqueous solution, with 20 % cyclodextrins as solubilisers, of one of the compounds of formula I mentioned in the preceding Examples 1 to 6 (e.g. Example 2) as active ingredient, is so mixed under aseptic conditions, with heating, with a sterile gelatine solution containing phenol as preservative, that 1.0ml of solution has the following composition:

| | |
|---|---|
| active ingredient | 3 mg |
| gelatine | 150.0 mg |
| phenol | 4.7 mg |
| dist. water with | 20 % cyclodextrins |
| as solubilisers | 1.0 ml |

Example 10: Sterile dry substance for injection:

5 mg of one of the compounds of formula I mentioned in the preceding Examples 1 to 6 (e.g. Example 4) as active ingredient are dissolved in 1 ml of an aqueous solution with 20 mg of mannitol and 20 % cyclodextrins as solubilisers. The solution is sterile-filtered and introduced under aseptic conditions into a 2 ml ampoule, deep-frozen and lyophilised. Before use, the lyophilisate is dissolved in 1 ml of distilled water or 1 ml of a physiological saline solution. The solution is administered intramuscularly or intravenously. This formulation can also be introduced into a twin-chambered injection ampoule.

Example 11: Nasal spray:

500 mg of finely ground (<5.0 mm) powder of one of the compounds of formula I men tioned in the preceding Examples 1 to 5 (e.g. Example 2) is suspended as active ingredient in a mixture of 3.5ml of Myglyol 812/ and 0.08 g of benzyl alcohol. The suspension is introduced into a container having a metering valve. 5.0 g of Freon 12/ are introduced under pressure into the container through the valve. The "Freon" is dissolved in the Myglyol/benzyl alcohol mixture by shaking. The spray container contains approximately 100 single doses which can be administered individually.

Example 12: Film-coated tablets

The following ingredients are used for the preparation of 10000 tablets each containing 100 mg of active ingredient:

| | |
|---|---|
| active ingredient | 1000 g |

(continued)

| | |
|---|---|
| corn starch | 680 g |
| colloidal silica | 200 g |
| magnesium stearate | 20 g |
| stearic acid | 50 g |
| sodium carboxymethyl starch | 250 g |
| water | quantum satis |

A mixture of one of the compounds of formula I mentioned in the preceding Examples 1 to 6 (e.g. Example 3) as active ingredient, 50 g of corn starch and the colloidal silica is processed with a starch paste, made from 250 g of corn starch and 2.2 kg of demineralised water, to form a moist mass. This is forced through a sieve having a mesh size of 3 mm and dried at 45° for 30min in a fluidised bed drier. The dry granules are pressed through a sieve having a mesh size of 1 mm, mixed with a pre-sieved mixture (1 mm sieve) of 330 g of corn starch, the magnesium stearate, the stearic acid and the sodium carboxymethyl starch, and compressed to form slightly biconvex tablets.

## Claims

**1.** A compound of formula I,

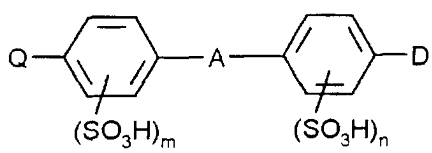

(I)

wherein

**A** is $-CH_2-CH_2-$ or -CH=CH-;

**D** is a radical of the partial formula (A),

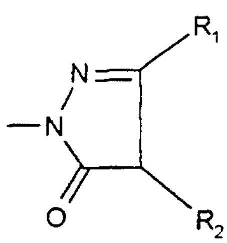

(A)

wherein
$R_1$ is hydrogen, lower alkyl, phenyl or carboxy; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group,
each of **m** and **n** is, independently of the other, 1, 2, 3 or 4; and

**Q** is selected from the group comprising

(a) amino,
(b) nitro,
(c) a radical of the partial formula (B),

-NH-G (B)

G is selected from the group comprising unsubstituted or oxo substituted lower alkanoyl; benzoyl; phenylaminocarbonyl wherein the phenyl is unsubstituted or substituted by (hydroxy and/or carboxy-substituted phenyl)azo; and naphthylaminocarbonyl wherein naphthyl is unsubstituted or substituted by one or more substituents selected from the group comprising hydroxy, sulfo, phenylazo and phenylazo wherein phenyl is substituted by one or more substituents independently selected from the group comprising hydroxy, carboxy, nitro or sulfo,

(d) a radical of any one of the partial formulae (C), (D) and (E)

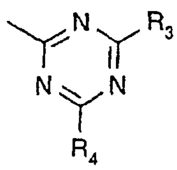

(C),

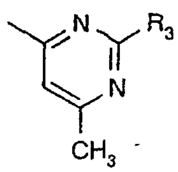

(D),

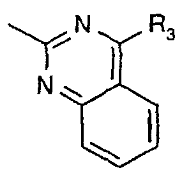

(E)

wherein

$R_3$ is selected from the group comprising

(i) a radical of the partial formula (F)

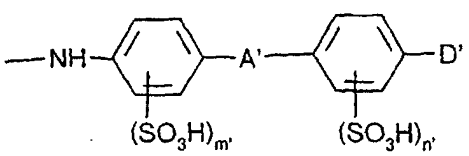

(F)

wherein

A' is $-CH_2-CH_2-$ or -CH=CH-,

D' is hydrogen, sulfo or a radical of the partial formula (A)

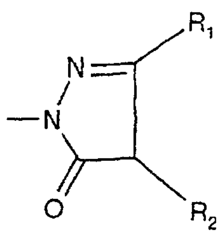

(A)

wherein

R1 is hydrogen, lower alkyl, phenyl or carboxy; and

R2 is hydrogen, phenylazo or naphthylazo wherein the phenyl or napthtyl is unsubstituted or substituted by one or more substituents selected from the group comprising halo, hydroxy, carboxy, nitro and sulfo,

and each of m' and n' is 1, 2, 3 or 4,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,

and

$R_4$ is phenyl, halo, amino, phenylamino, hydroxyphenylamino or (hydroxy and/or sulfo-substituted phenyl) azo-phenylamino,

(e) a radical of the partial formula (G)

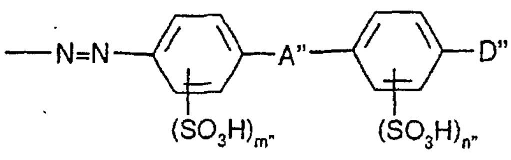

(G)

wherein

A" is $-CH_2-CH_2-$ or -CH=CH-,

D" is a radical of the partial formula (A)

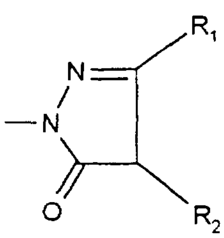

(A)

wherein

R1 is hydrogen, lower alkyl, phenyl or carboxy; and

R2 is hydrogen, phenylazo or naphthylazo wherein the phenyl or napthtyl is unsubstituted or substituted by one or more substituents selected from the group comprising halo, hydroxy, carboxy, nitro and sulfo,

and each of m" and n" is 1, 2, 3 or 4,

(f) a radical of the partial formula (H)

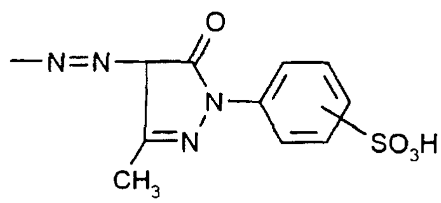

(H);

and

(g) a radical of the partial formula (K),

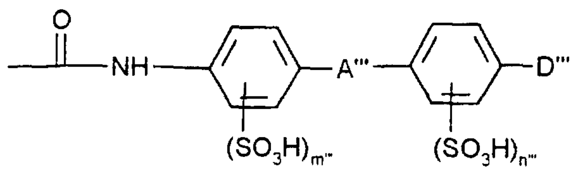

wherein

A''' is $-CH_2-CH_2-$ or -CH=CH-;

D''' is a radical of the partial formula (A),

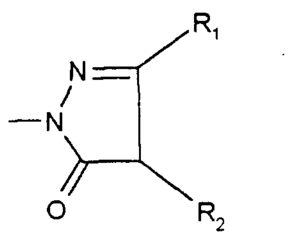

(A)

wherein

$R_1$ is hydrogen, lower alkyl, phenyl or carboxy; and

$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group, and

each of m''' and n''' is 1, 2, 3 or 4;

or a tautomer thereof; or a salt or a complex salt thereof where at least one salt-forming or complex-forming group is present;
for use in the treatment of the animal, including human, body.

**2.** A compound of formula I according to claim 1 for use in the treatment of the animal, including human, body, wherein

**A** is -CH=CH-, preferably in the trans configuration;

**D** is a radical of the partial formula (A),

wherein

$R_1$ is hydrogen, lower alkyl, especially methyl, phenyl or carboxy, preferably methyl; and

$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitrophenylazo, 2-hydroxy-5-sulfo-phenylazo, 2-hydroxy-4-sulfo-1-naphthylazo and especially 2-carboxy-phenylazo;

each of m and n is 1, the corresponding sulfo group being preferably bonded in the o-position with regard to

the bivalent radical -A- in formula I; and
**Q** is selected from the group consisting of

(i) amino,
(ii) a radical of the partial formula (B), wherein G is selected from the group comprising unsubstituted or oxo substituted lower alkanoyl, especially acetoacetyl; a radical of the partial formula (C)
wherein
$R_3$ is a radical of the partial formula (F)
wherein
A' is -CH=CH-, preferably in the trans-configuration,
D' is hydrogen, sulfo or preferably a radical of the partial formula (A)
wherein
$R_1$ is hydrogen, lower alkyl, especially methyl, phenyl or carboxy, preferably methyl; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitrophenylazo, 2-hydroxy-5-sulfo-phenylazo, 2-hydroxy-4-sulfo-1-naphthylazo and especially 2-carboxy-phenylazo;
and each of m' and n' is 1, the corresponding sulfo group being preferably bonded in the o-position with regard to the bivalent radical -A- in formula I;
and
$R_4$ is halo, especially chloro, or amino,
and
(iii) a radical of the partial formula (K)
wherein
A''' is -CH=CH-, preferably in the trans-configuration,
D''' is hydrogen, sulfo or a radical of the partial formula (A) wherein
$R_1$ is hydrogen, phenyl, carboxy or preferably methyl; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group; especially selected from the group consisting of hydrogen, 5-chloro-2-hydroxy-phenylazo, 2-hydroxy-4-nitro-phenylazo, 2-hydroxy-5-sulfo-phenylazo, 2-hydroxy-4-sulfo-1-naphthylazo and especially 2-carboxy-phenylazo;
and each of m''' and n''' is 1, the corresponding sulfo group being preferably bonded in the o-position with regard to the bivalent radical -A'''- in formula I,

or a tautomer thereof; or a pharmaceutically acceptable salt or a pharmaceutically acceptable complex salt thereof where at least one salt-forming or complex-forming group is present.

**3.** A compound according to claim 1 for use in the treatment of the animal, including human, body, of the formula I selected from the group consisting of
trans-5-amino-2-[2-{4-(4,5-dihydro-3-methyl-5-oxo-1H-pyrazol-1-yl)-2-sulfophenyl]ethenyl]-benzenesulfonic acid;
N,N'-bis(trans-4-{2-[4-(3-methyl-5-oxo-4-(o-carboxyphenylazo)-4,5-dihydro-1.H.-pyrazol-1-yl)-2-sulfo-phenyl]-vinyl}-2-sulfophenyl)-urea; and
N, N'-bis(trans-4-{2-[4-(3-methyl-5-oxo-4-(o-carboxyphenylazo)-4,5-dihydro-1.H.-pyrazol-1-yl)-2-sulfo-phenyl]-vinyl}-2-sulfophenyl)-urea copper complex,
or a pharmaceutically acceptable salt thereof.

**4.** A compound according to claim 1 for use in the treatment of the animal, including human, body, of the formula I named trans-5-(acetoacetylamino)-2-[2-{4-(4,5-dihydro-3-methyl-5-oxo-1H-pyrazol-1-yl)-2-sulfophenyl}ethenyl]-benzenesulfonic acid; or a pharmaceutically acceptable salt thereof.

**5.** The use of a compound of formula I, a tautomer thereof, a salt or a complex salt thereof as shown in claim 1 for the manufacture of a pharmaceutical preparation for the inhibition of the interaction of a transcriptional regulator with a retroviral response element or of additional specific functions of Tat, such as specific activation of cellular HIV-promoting processes, especially expression of cellular coreceptors for HIV, especially CXCR4.

**6.** The use of a compound of formula I, a tautomer thereof, a salt or a complex salt thereof as shown in claim 1 for the manufacture of a pharmaceutical preparation for the treatment of a virus disease.

**7.** The use of a compound of formula I, a tautomer thereof, a salt or a complex salt thereof as shown in claim 1 for the manufacture of a pharmaceutical preparation useful as an antiretroviral therapeutic inhibiting the interaction of a transcriptional regulator with a retroviral response element or inhibiting additional specific functions of Tat, such as activation of cellular HIV-promoting processes, especially expression of cellular coreceptors for HIV, especially CXCR4,.

**8.** A compound of formula I,

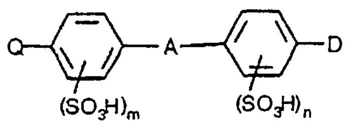

(I)

wherein

**A** is $-CH_2-CH_2-$ or $-CH=CH-$;

**D** is a radical of the partial formula (A),

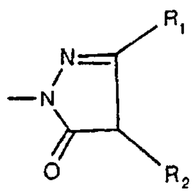

(A)

wherein
$R_1$ is hydrogen, lower alkyl, phenyl or carboxy; and
$R_2$ is hydrogen, phenylazo or naphthylazo wherein the phenyl or naphthtyl is unsubstituted or substituted by one or more substituents selected from the group comprising halo, hydroxy, carboxy, nitro and sulfo, with at least one hydroxy or carboxy substituent in ortho-position to the azo group,
each of **m** and **n** is 1, 2, 3 or 4; and

**Q** is a radical of of the partial formulae (C),

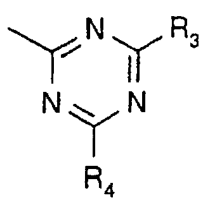

(C),

wherein
$R_3$ is selected from the group comprising

(i) a radical of the partial formula (F),

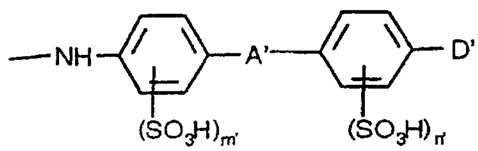

(F)

wherein
A' is $-CH_2-CH_2-$ or -CH=CH-,
D' is hydrogen, sulfo or a radical of the partial formula (A)
wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo,
and each of m' and n' is 1, 2, 3 or 4, preferably 1,

(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and

(iii) naphthylamino wherein naphthyl is substituted by up to four radicals selected from the group comprising hydroxy, sulfo, (hydroxy and/or chloro-substituted phenyl)azo, (carboxy-substituted phenyl)azo and (hydroxy and/or nitro-substituted phenyl)azo,

and
$R_4$ is amino,

or a tautomer thereof; or a salt or a complex salt thereof where at least one salt-forming or complex-forming group is present.

**9.** A compound of the formula I according to claim 8, wherein

A is -CH=CH-, especially in the trans configuration;
D is a radical of the partial formula (A) wherein
$R_1$ is lower alkyl, and
$R_2$ is hydrogen,
each of m and n is 1, the corresponding sulfo group being preferably bonded in the o-position with regard to the bivalent radical -A- in formula I; and
Q is a radical of the partial formula (C),
wherein
$R_3$ is a radical of the partial formula (F),
wherein
A' is -CH=CH-, especially in the trans configuration,
D' is a radical of the partial formula (A)
wherein
R1 is lower alkyl, especially methyl; and
R2 is hydrogen,
each of m and n is 1, the corresponding sulfo group being preferably bonded in the o-position with regard to the bivalent radical -A- in formula I;
and
$R_4$ is amino,

or a tautomer thereof; or a salt or a complex salt thereof where at least one salt-forming or complex-forming group is present.

**10.** A compound of formula I named N,N'-bis(trans-4-{2-[4-(3-methyl-5-oxo-4,5-dihydro-1.H.-pyrazol-1-yl)-2-sulfophenyl]-vinyl}-2-sulfophenyl)-[1,3,5]triazin-2,4,6-triamine or N, N'-bis(trans-4-{2-[4-(3-methyl-5-oxo-4,5-dihydro-1.H.-

pyrazol-1-yl}-2-sulfo-phenyl]-vinyl}-2-sulfophenyl)-2-chloro-[1,3,5]triazine-4,6-diamine;
or a tautomer thereof; or a pharmaceutically acceptable salt thereof.

**11.** A compound of formula I, or a tautomer thereof; or a salt or a complex salt thereof where at least one salt-forming or complex-forming group is present according to any one of claims 8 to 10 for for use in the treatment of the animal, including human, body.

**12.** A pharmaceutical preparation comprising a compound of formula I, or a tautomer thereof; or a salt or a complex salt thereof where at least one salt-forming or complex-forming group is present, according to any one of claims 8 to 10 and a pharmaceutically acceptable carrier.

**13.** The use of a compound of formula I, or a tautomer thereof; or a salt or a complex salt thereof where at least one salt-forming or complex-forming group is present, according to claim 1, for the manufacture of a pharmaceutical preparation useful as an antiretroviral therapeutic inhibiting the interaction of a transcriptional regulator with a retroviral response element or inhibiting additional specific functions of Tat, such as activation of cellular HIV-promoting processes, especially expression of cellular coreceptors for HIV, especially CXCR4.

**14.** A process for the preparation of a compound of the formula I, or a tautomer thereof; or a salt or a complex salt thereof where at least one salt-forming or complex-forming group is present, according to claim 8, in which a compound of the formula II,

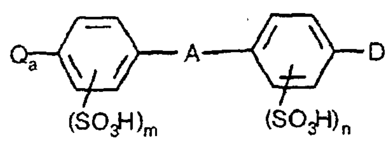

(II)

wherein

$Q_a$ is a radical of the partial formula (C),

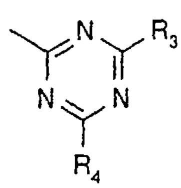

(C),

wherein
$R_3$ is selected from the group comprising

(i) a radical of the partial formula (F),
wherein
A' is $-CH_2-CH_2-$ or -CH=CH-,
D' is hydrogen, sulfo or a radical of the partial formula (A)
wherein
R1 is hydrogen, lower alkyl, phenyl or carboxy; and
R2 is hydrogen, phenylazo or naphthylazo wherein the phenyl or napthtyl is unsubstituted or substituted by one or more substituents selected from the group comprising halo, hydroxy, carboxy, nitro and sulfo,
and each of m' and n' is 1, 2, 3 or 4,
(ii) phenylamino wherein phenyl is subsituted by sulfophenylazo, by (hydroxy and/or carboxy)phenylazo, by methyl and 3-methyl-4-[(mono- or di-sulfo)naphthylazo-]-phenylazo or by [2-(phenylazo)-2-(acetyl)-acetyl]amino; and
(iii) naphthylamino wherein naphthyl is substituted by up to four radicals selected from the group comprising hydroxy, sulfo, (hydroxy and/or chloro-substituted phenyl)azo, (carboxy-substituted phenyl)azo and

(hydroxy and/or nitro-substituted phenyl)azo,

and
$R_4$ is halo, and
and A, D, m and n have the meanings given for a compound of formula I in claim 7, is reacted with ammonia and, if desired, a free compound of formula I or a tautomer thereof obtainable according to this process is converted into a salt or a complex salt, an obtainable salt or a complex salt of a compound of formula I or a tautomer thereof is converted into a different salt or complex salt or into a free compound of formula I or a tautomer thereof, and/or a mixture of isomers of an obtained compound of formula I is separated into individual isomers.

**Patentansprüche**

**1.** Verbindung der Formel I,

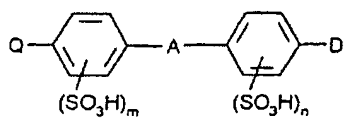

(I)

wobei

A $-CH_2-CH_2-$ oder -CH=CH-,

D einen Rest der Teilformel (A),

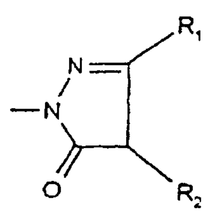

(A)

wobei
$R_1$ für Wasserstoff, niederes Alkyl, Phenyl oder Carboxy, und
$R_2$ für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, wobei sich wenigstens ein Hydroxyoder Carboxy-Substituent in ortho-Stellung zur Azogruppe befindet, substituiert ist, bedeutet,
m und n jeweils unabhängig voneinander 1, 2, 3 oder 4 ist; und

Q ausgewählt ist aus der Gruppe bestehend aus

(a) Amino,

(b) Nitro,

(c) einem Rest der Teilformel (B),

-NH-G (B)

wobei

G ausgewählt ist aus der Gruppe bestehend aus gegebenenfalls oxo-substituiertem niederem Alkanoyl; Benzoyl; Phenylaminocarbonyl, wobei das Phenyl gegebenenfalls mit (hydroxyund/oder carboxy-substituiertem Phenyl)-Azo substituiert ist, und Naphthylaminocarbonyl, wobei Naphthyl gegebenenfalls mit einem oder mehreren Substituenten, ausgewählt aus der Gruppe bestehend aus Hydroxy, Sulfo, Phenylazo und Phenylazo, wobei Phenyl mit einem oder mehreren unabhängig aus der Gruppe bestehend aus Hydroxy, Carboxy, Nitro oder Sulfo ausgewählten Substituenten substituiert ist, substituiert ist,

(d) einem Rest einer der Teilformeln (C), (D) und (E)

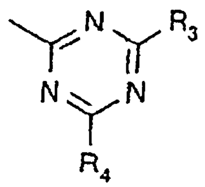

(C),

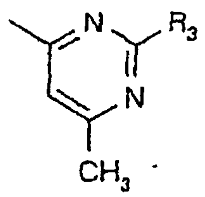

(D),

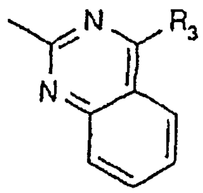

(E),

wobei
$R_3$ ausgewählt ist aus der Gruppe bestehend aus

(i) einem Rest der Teilformel (F)

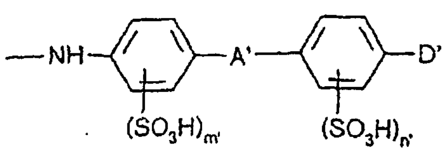

(F)

wobei
A' $-CH_2-CH_2-$ oder -CH=CH-,
D' Wasserstoff, Sulfo oder einen Rest der Teilformel (A)

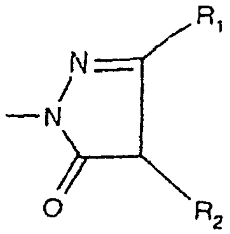

(A)

wobei

R1 für Wasserstoff, niederes Alkyl, Phenyl oder Carboxy, und

R2 für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, substituiert ist, bedeutet, und m' und n' jeweils 1, 2, 3 oder 4 ist,

(ii) Phenylamino, wobei Phenyl mit Sulfophenylaz o, mit (Hydroxy- und/oder Carboxy-)phenylazo, mit Methyl und 3-Methyl-4-[(mono- oder di-sulfo)-naphthylazo-]phenylazo oder mit [2-(Phenylazo)-2-(acetyl)acetyl]amino substituiert ist, und

(iii) Naphthylamino, wobei Naphthyl mit bis zu vier Resten, ausgewählt aus der Gruppe bestehend aus Hydroxy, Sulfo, (hydroxy- und/oder chlor-substituiertem Phenyl)azo, (carboxy-substituiertem Phenyl)azo und (hydroxy- und/oder nitro-substituiertem Phenyl)azo, substituiert ist,

und

$R_4$ für Phenyl, Halogen, Amino, Phenylamino, Hydroxyphenylamino oder (hydroxy- und/oder sulfosubstituiertes Phenyl)azo-phenylamino steht,

(e) einem Rest der Teilformel (G)

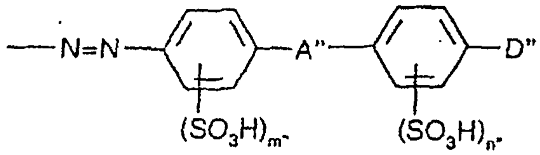

(G)

wobei

A" $-CH_2-CH_2-$ oder -CH=CH-,

D" einen Rest der Teilformel (A)

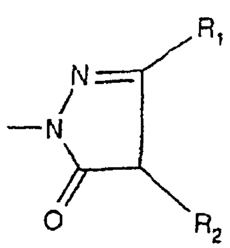

(A)

wobei

R1 für Wasserstoff, niede 'res Alkyl, Phenyl oder Carboxy, und

R2 für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, substituiert ist, bedeutet,

und m" und n" jeweils 1, 2, 3 oder 4 ist,

(f) einem Rest der Teilformel (H)

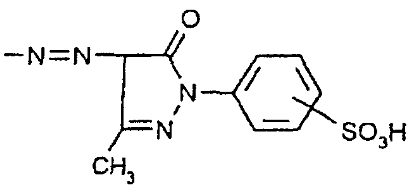

(H)

und

(g) einem Rest der Teilformel (K),

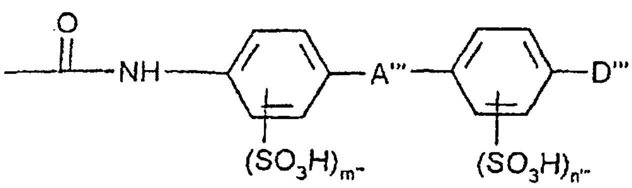

wobei
A'''-$CH_2$-$CH_2$- oder -CH=CH-,
D'''einen Rest der Teilformel (A)

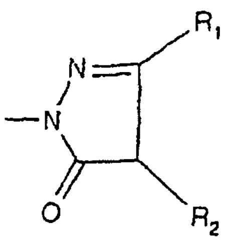

(A)

wobei
$R_1$ für Wasserstoff, niederes Alkyl, Phenyl oder Carboxy, und
$R_2$ für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, wobei sich wenigstens ein Hydroxy- oder Carboxysubstituent in ortho-Stellung zur Azogruppe befindet, substituiert ist, bedeutet,
und m'''und n'''jeweils 1, 2, 3 oder 4 ist,

oder ein Tautomer davon, oder ein Salz oder Komplexsalz davon, in dem wenigstens eine salzbildende oder komplexbildende Gruppe vorhanden ist,

zur Verwendung bei der Behandlung des tierischen, einschließlich menschlichen, Organismus.

**2.** Verbindung der Formel I nach Anspruch 1 bei der Behandlung des tierischen, einschließlich menschlichen, Organismus, wobei

A -CH=CH-, vorzugsweise in trans-Konfiguration,
D einen Rest der Teilformel (A),
wobei

$R_1$ für Wasserstoff, niederes Alkyl, insbesondere Methyl, Phenyl oder Carboxy, vorzugsweise Methyl, und

$R_2$ für Wasserstoff, Phenylazo oder Naphthylazo steht, wobei das Phenyl oder Naphthyl mit einem oder mehreren Substituenten, ausgewählt aus der Gruppe bestehend aus Halogen, Hydroxy, Carboxy, Nitro und Sulfo, wobei sich wenigstens ein Hydroxy- oder Carboxy-Substituent in ortho-Stellung zur Azogruppe befindet, insbesondere ausgewählt aus der Gruppe bestehend aus Wasserstoff, 5-Chlor-2-hydroxy-phenylazo, 2-Hydroxy-4-nitrophenylazo, 2-Hydroxy-5-sulfophenylazo, 2-Hydroxy-4-sulfo-1-naphthylazo und insbesondere 2-Carboxy-phenylazo, substituiert ist, bedeutet,

m und n jeweils 1 ist, wobei die entsprechende Sulfogruppe vorzugsweise in o-Stellung bezüglich des zweiwertigen Rests -A- in Formel I gebunden ist, und

Q ausgewählt ist aus der Gruppe bestehend aus

(i) Amino,

(ii) einem Rest der Teilformel (B), wobei G ausgewählt ist aus der Gruppe bestehend aus gegebenenfalls oxo-substituiertem niederem Alkanoyl, insbesondere Acetoacetyl; einem Rest der Teilformel (C),

wobei

$R_3$ für einen Rest der Teilformel (F), wobei

A' -CH=CH-, vorzugsweise in trans-Konfiguration,

D' Wasserstoff, Sulfo oder vorzugsweise einen Rest der Teilformel (A),

wobei

$R_1$ für Wasserstoff, niederes Alkyl, insbesondere Methyl, Phenyl oder Carboxy, vorzugsweise Methyl, und

$R_2$ für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, wobei sich wenigstens ein Hydroxy- oder Carboxy-Substituent in ortho-Stellung zur Azogruppe befindet, insbesondere ausgewählt aus der Gruppe bestehend aus Wasserstoff, 5-Chlor-2-hydroxyphenylazo, 2-Hydroxy-4-nitro-phenylazo, 2-Hydroxy-5-sulfo-phenylazo, 2-Hydroxy-4-sulfo-1-naphthylazo und insbesondere 2-Carboxy-phenylazo, substituiert ist, bedeutet,

und m' und n' jeweils 1 ist, wobei die entsprechende Sulfogruppe vorzugsweise in o-Stellung bezüglich des zweiwertigen Rests -A- in Formel I gebunden ist,

und

$R_4$ für Halogen, insbesondere Chlor, oder Amino steht,

und

(iii) einem Rest der Teilformel (K)

wobei

A'''-CH=CH-, vorzugsweise in trans-Konfiguration,

D'''Wasserstoff, Sulfo oder einen Rest der Teilformel (A), wobei

$R_1$ für Wasserstoff, Phenyl, Carboxy oder vorzugsweise Methyl, und

$R_2$ für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, wobei sich wenigstens ein Hydroxy- oder Carboxysubstituent in ortho-Stellung zur Azogruppe befindet, insbesondere ausgewählt aus der Gruppe bestehend aus Wasserstoff, 5-Chlor-2-hydroxy-phenylazo, 2-Hydroxy-4-nitro-phenylazo, 2-Hydroxy-5-sulfo-phenylazo, 2-Hydroxy-4-sulfo-1-naphthylazo und insbesondere 2-Carboxy-phenylazo, substituiert ist, bedeutet,

und m '''und n '''jeweils 1 ist, wobei die entsprechende Sulfogruppe vorzugsweise in o-Stellung bezüglich des zweiwertigen Rests -A''' in Formel I gebunden ist,

oder ein Tautomer davon, od er ein pharmazeutisch unbedenkliches Salz oder ein pharmazeutisch unbedenkliches Komplexsalz davon, in dem wenigstens eine salz bildende oder komplexbildende Gruppe vorhanden ist.

**3.** Verbindung nach Anspruch 1 zur Verwendung bei der Behandlung des tierischen, einschließlich menschlichen, Organismus, wobei es sich um eine Verbindung der Formel I, ausgewählt aus der Gruppe bestehend aus trans-5-Amino-2-[2-{4-(4,5-dihydro-3-methyl-5-oxolH-pyrazol-l-yl)-2-sulfophenyl}ethenyl]benzolsulfonsäure, N,N'-Bis(trans-4-{2-[4-(3-methyl-5-oxo-4-(ocarboxyphenylazo)-4,5-dihydro-l.H.-pyrazol-1-yl)-2-sulfophenyl] vinyl}-2-sulfophenyl)harnstoff, und N,N'-Bis(trans-4-{2-[4-(3-methyl-5-oxo-4-(ocarboxyphenylazo)-4,5-dihydro-l.H.-pyrazol-1-yl)-2-sulfophenyl] vinyl}-2-sulfophenyl)harnstoffkupfer-Komplex, handelt, oder ein pharmazeutisch unbedenkliches Salz davon.

**4.** Verbindung nach Anspruch 1 zur Verwendung bei der Behandlung des tierischen, einschließlich menschlichen, Organismus, wobei es sich um eine Verbindung der Formel I mit der Bezeichnung trans-5-(Acetoacetylamino)-2-[2-{4-(4,5-dihydro-3-methyl-5-oxo-1H-pyrazol-1-yl)-2-sulfophenyl}ethenyl]benzolsulfonsäure handelt oder ein pharmazeutisch unbedenkliches Salz davon.

**5.** Verwendung einer Verbindung der For mel I, eines Tautomers davon, eines Salzes oder Komplexsalzes davon mit der in Anspruch 1 angegebenen Bedeutung zur Herstellung einer pharmazeutischen Zubereitung zur Hemmung der Wechselwirkung eines Transkriptionsregulators mit einem RRE (Retroviral Response Element)-Element oder zusätzlicher spezifischer Tat-Funktionen, wie beispielsweise der spezifischen Aktivierung zellulärer, HIV-begünstigender Vorgänge, insbesondere der Expression zellulärer Corezeptoren für HIV, insbesondere CXCR4.

**6.** Verwendung einer Verbindung der Formel I, eines Tautomers davon, eines Salzes oder Komplexsalzes davon mit der in Anspruch 1 angegebenen Bedeutung zur Herstellung einer pharmazeutischen Zubereitung zur Behandlung einer Viruserkrankung.

**7.** Verwendung einer Verbindung de r Formel I, eines Tautomers davon, eines Salzes oder Komplexsalzes davon mit der in Anspruch 1 angegebenen Bedeutung zur Herstellung einer als antiretrovirales Therapeutikum geeigneten pharmazeutischen Zubereitung, mit dem die Wechselwirkung eines Transkriptionsregulators mit einem RRE-Element oder zusätzliche spezifische Tat-Funktionen, wie beispielsweise die Aktivierung zellulärer, HIV-begünstigender Vorgänge, insbesondere der Expression zellulärer Corezeptoren für HIV, insbesondere CXCR4, inhibiert werden.

**8.** Verbindung der Formel I,

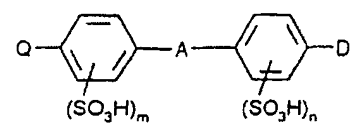

(I)

wobei

A $-CH_2-CH_2-$ oder -CH=CH-,

D einen Rest der Teilformel (A),

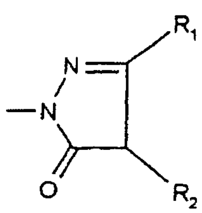

(A)

wobei

$R_1$ für Wasserstoff, niederes Alkyl, Phenyl oder Carboxy, und

$R_2$ für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, wobei sich wenigstens ein Hydroxyoder Carboxy-Substituent in ortho-Stellung zur Azogruppe befindet, substituiert ist, bedeutet,

m und n jeweils 1, 2, 3 oder 4 ist; und

Q einen Rest der Teilformel (C),

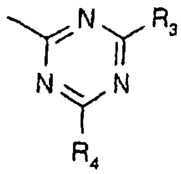

(C),

wobei
$R_3$ ausgewählt ist aus der Gruppe bestehend aus

(i) einem Rest der Teilformel (F)

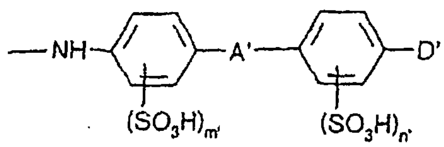

(F)

wobei
A' $-CH_2-CH_2-$ oder -CH=CH-,
D' Wasserstoff, Sulfo oder einen Rest der Teilformel (A)
wobei
R1 für Wasserstoff, niederes Alkyl, Phenyl oder Carboxy, und
R2 für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, substituiert ist, bedeutet,
und m ' und n' jeweils 1, 2, 3 oder 4, vorzugsweise 1, ist,

(ii) Phenylamino, wobei Phenyl mit Su lfophenylazo, mit (Hydroxy- und/oder Carboxy-)phenylazo, mit Methyl und 3-Methyl-4-[(mono- oder di-sulfo)-naphthylazo-]phenylazo oder mit [2-(Phenylazo)-2-(acetyl)acetyl]amino substituiert ist, und

(iii) Naphthylamino, wobei Naphthyl mit bis zu vier Resten, ausgewählt aus der Gruppe bestehend aus Hydroxy, Sulfo, (hydroxy- und/oder chlor-substituiertem Phenyl)azo, (carboxy-substituiertem Phenyl)azo und (hydroxy- und/oder nitro-substituiertem Phenyl)azo, substituiert ist,

und
$R_4$ für Amino steht,

oder e in Tautomer davon, oder ein Salz oder Komplexsalz davon, in dem wenigstens eine salzbildende oder komplexbildende Gruppe vorhanden ist.

**9.** Verbindung der Formel I nach Anspruch 8, wobei

A -CH=CH-, insbesondere in trans-Konfiguration,
D einen Rest der Teilformel (A), wobei
$R_1$ für niederes Alkyl und
$R_2$ für Wasserstoff steht, bedeutet,
m und n jeweils 1 ist, wobei die entsprechende Sulfogruppe vorzugsweise in o-Stellung bezüglich des zweiwertigen Rests -A- in Formel I gebunden ist, und
Q einen Rest der Teilformel (C) bedeutet,
wobei
$R_3$ für einen Rest der Teilformel (F),
wobei
A' -CH=CH-, insbesondere in trans-Konfiguration,

D' einen Rest der Teilformel (A),
wobei
R1 für niederes Alkyl, insbesondere Methyl, und
R2 für Wasserstoff steht, bedeutet,
und m und n jeweils 1 ist, wobei die entsprechende Sulfogruppe vorzugsweise in o-Stellung bezüglich des zweiwertigen Rests -Ain Formel I gebunden ist,
und
$R_4$ für Amino steht,

oder ein Tautomer davon, oder ein Salz oder Komplexsalz davon, in dem wenigstens eine salzbildende oder komplexbildende Gruppe vorhanden ist.

**10.** Verbindung der Formel I mit der Bezeichnung N,N'-Bis(trans-4-{2-[4-(3-methyl-5-oxo-4,5-dihydro-l.H.-pyrazol-1-yl)-2-sulfophenyl]vinyl}-2-sulfophenyl)-[1,3,5]triazin-2,4,6-triamin oder N,N'-Bis(trans-4-{2-[4-(3-methyl-5-oxo-4,5-dihydro-1.H.-pyrazol-1-yl)-2-sulfophenyl]vinyl}-2-sulfophenyl)-2-chlor-[1,3,5]triazin-4,6-diamin, oder ein Tautomer davon oder ein pharmazeutisch unbedenkliches Salz davon.

**11.** Verbindung der Formel I oder ein Tautomer davon oder ein Salz oder ein Komplexsalz davon, in dem wenigstens eine salzbildende oder komplexbildende Gruppe vorhanden ist, nach einem der Ansprüche 8 bis 10 zur Verwendung bei der Behandlung des tierischen, einschließlich menschlichen, Organismus.

**12.** Pharmazeutische Zubereitung, enthaltend eine Verbindung der Formel I oder ein Tautomer davon oder ein Salz oder ein Komplexsalz davon, in dem wenigstens eine salzbildende oder komplexbildende Gruppe vorhanden ist, nach einem der Ansprüche 8 bis 10 und einen pharmazeutisch unbedenklichen Trägerstoff.

**13.** Verwendung einer Verbindung der Formel I oder eines Tautomers davon oder eines Salzes oder eines Komplexsalzes davon, in dem wenigstens eine salzbildende oder komplexbildende Gruppe vorhanden ist, nach Anspruch 1 zur Herstellung einer als antiretrovirales Therapeutikum geeigneten pharmazeutischen Zubereitung, mit dem die Wechselwirkung eines Transkriptionsregulators mit einem RRE-Element oder zusätzliche spezifische Tat-Funktionen, wie beispielsweise die Aktivierung zellulärer, HIV -begünstigender Vorgänge, insbesondere der Expression zellulärer Corezeptoren für HIV, insbesondere CXCR4, gehemmt werden.

**14.** Verfahren zur Herstellung einer Verbindung der Formel I oder eines Tautomers davon oder eines Salzes oder eines Komplexsalzes davon, in dem wenigstens eine salzbildende oder komplexbildende Gruppe vorhanden ist, nach Anspruch 8, bei dem eine Verbindung der Formel II,

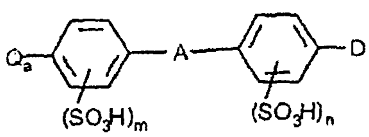

(II)

wobei

$Q_a$ einen Rest der Teilformel (C)

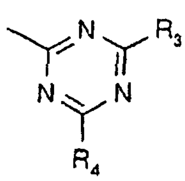

(C)

bedeutet, wobei
$R_3$ ausgewählt ist aus der Gruppe bestehend aus

(i) einem Rest der Teilformel (F),
wobei
A' $-CH_2-CH_2-$ oder -CH=CH-,
D' Wasserstoff, Sulfo oder einen Rest der Teilformel (A),
wobei
R1 für Wasserstoff, niederes Alkyl, Phenyl oder Carboxy, und
R2 für Wasserstoff, Phenylazo oder Naphthylazo steht, wobei das Phenyl oder Naphthyl gegebenenfalls mit einem oder mehreren Substituenten, ausgewählt aus der Gruppe bestehend aus Halogen, Hydroxy, Carboxy, Nitro und Sulfo, substituiert ist, bedeutet,
und m' und n' jeweils 1, 2, 3 oder 4 ist,

(ii) Phenylamino, wobei Phenyl mit Sulfophenylazo, mit (Hydroxy- und/oder Carboxy-)phenylazo, mit Methyl und 3-Methyl-4-[(mono- oder di-sulfo) naphthylazo-]phenylazo oder mit [2-(Phenylazo)-2-(acetyl)acetyl]amino substituiert ist, und

(iii) Naphthylamino, wobei Naphthyl mit bis zu vier Resten, ausgewählt aus der Gruppe bestehend aus Hydroxy, Sulfo, (hydroxyund/oder chlor-substituiertem Phenyl)azo, (carboxy-substituiertem Phenyl)azo und (hydroxy- und/oder nitro-substituiertem Phenyl)azo, substituiert ist,
und
$R_4$ für Halogen steht,

und A, D, m und n die bei einer Verbindung der Formel I in Anspruch 7 angegebenen Bedeutungen besitzen, mit Ammoniak umgesetzt wird,

und gewünschtenfalls eine freie Verbindung der Formel I oder ein gemäß diesem Verfahren erhältliches Tautomer davon in ein Salz oder ein Komplexsalz umgewandelt wird, ein erhältliches Salz oder ein Komplexsalz einer Verbindung der Formel I oder eines Tautomers davon in ein anderes Salz oder Komplexsalz oder in eine freie Verbindung der Formel I oder eines Tautomers davon umgewandelt wird und/oder ein Isomerengemisch einer erhaltenen Verbindung der Formel I in einzelne Isomere getrennt wird.

## Revendications

**1.** Composé de formule I,

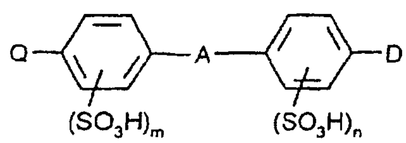

(I)

dans laquelle

A est $-CH_2-CH_2-$ ou -CH=CH-;
D est un radical de formule partielle (A),

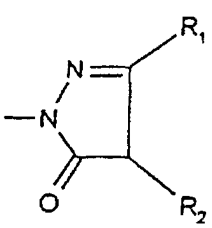

(A)

dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo,
chacun de m et n est, indépendamment l'un de l'autre, 1, 2, 3 ou 4; et
Q est sélectionné parmi le groupe comprenant

(a) un amino,
(b) un nitro,
(c) un radical de formule partielle (B),

-NH-G (B)

dans laquelle
G est sélectionné parmi le groupe comprenant un alcanoyle inférieur non substitué ou substitué par un oxo; un benzoyle; un phénylaminocarbonyle dans lequel le phényle est non substitué ou substitué par un (phényl substitué par un hydroxy et/ou un carboxy)azo; et un naphtylaminocarbonyle dans lequel le naphtyle est non substitué ou substitué par un ou plusieurs substituants sélectionnés parmi le groupe comprenant un hydroxy, un sulfo, un phénylazo et un phénylazo dans lequel le phényle est substitué par un ou plusieurs substituants sélectionnés indépendamment parmi le groupe comprenant un hydroxy, un carboxy, un nitro ou un sulfo,
(d) un radical suivant l'une quelconque des formules partielles (C), (D) et (E)

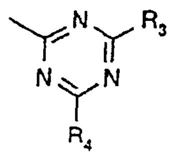
(C),

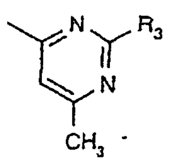
(D),

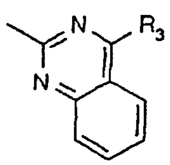
(E)

dans lesquelles
$R_3$ est sélectionné parmi le groupe comprenant

(i) un radical de formule partielle (F)

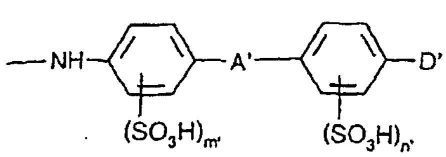
(F)

dans laquelle

A' est $-CH_2-CH_2-$ ou -CH=CH-,
D' est un hydrogène, un sulfo ou un radical de formule partielle (A)

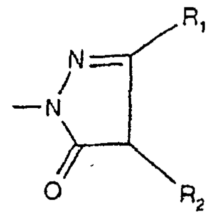

(A)

dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo,
et chacun de m' et n' est 1, 2, 3 ou 4,
(ii) un phénylamino dans lequel le phényle est substitué par un sulfophénylazo, par un (hydroxy et/ou carboxy)phénylazo, par un méthyle et un 3-méthyl-4-[(mono- ou disulfo)naphtylazo]phénylazo ou par un [2-(phénylazo)-2-(acétyl)acétyl]amino; et
(iii) un naphtylamino dans lequel le naphtyle est substitué par jusqu'à quatre radicaux sélectionnés parmi le groupe comprenant un hydroxy, un sulfo, un (phényl substitué par un hydroxy et/ou un chloro) azo, un (phényl substitué par un carboxy)azo et un (phényl substitué par un hydroxy et/ou un nitro) azo, et
$R_4$ est un phényle, un halogéno, un amino, un phénylamino, un hydroxyphénylamino ou un (phényl substitué par un hydroxy et/ou un sulfo)azophénylamino,

(e) un radical de formule partielle (G)

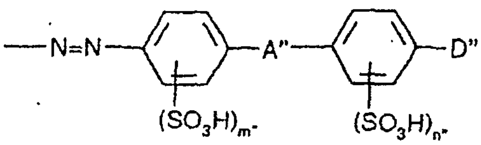

(G)

dans laquelle
A'' est $-CH_2-CH_2-$ ou -CH=CH-,
D'' est un radical de formule partielle (A)

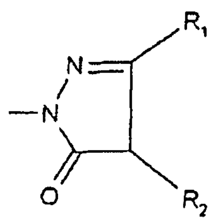

(A)

dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo,
et chacun de m'' et n'' est 1, 2, 3 ou 4,
(f) un radical de formule partielle (H)

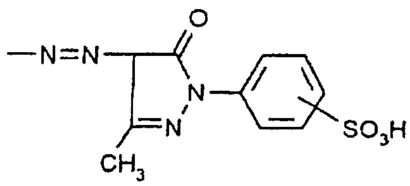

(H);

et

(g) un radical de formule partielle (K),

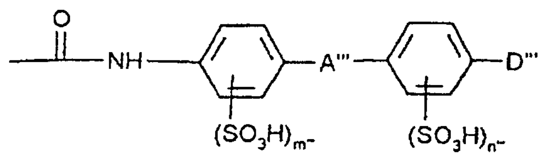

dans laquelle

A''' est $-CH_2-CH_2-$ ou -CH=CH-;

D''' est un radical de formule partielle (A),

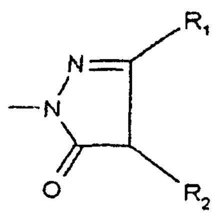

(A)

dans laquelle

$R_1$ est un hydrogène, un alkyle inférieur, un phényle ou un carboxy; et

$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo, et

chacun de m''' et n''' est 1, 2, 3 ou 4;

ou un tautomère de celui-ci; ou un sel ou un sel complexe de celui-ci où au moins un groupement formant un sel ou formant un complexe est présent;

pour une utilisation dans le traitement d'un animal, notamment l'homme.

**2.** Composé de formule I suivant la revendication 1, pour une utilisation dans le traitement d'un animal, notamment l'homme, dans laquelle

A est -CH=CH-, de préférence en configuration trans;

D est un radical de formule partielle (A),

dans laquelle

$R_1$ est un hydrogène, un alkyle inférieur, spécialement un méthyle, un phényle ou un carboxy, de préférence un méthyle; et

$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo; spécialement sélectionné parmi le groupe comprenant un hydrogène, un 5-chloro-2-hydroxyphénylazo, un 2-hydroxy-4-nitrophénylazo, un 2-hydroxy-5-sulfophénylazo, un 2-hydroxy-4-sulfo-1-naphtylazo et spécialement un 2-carboxyphénylazo; chacun de m et n est 1, le groupement sulfo correspondant étant de préférence lié en position o par rapport au radical bivalent -A- dans la formule I; et

Q est sélectionné parmi le groupe comprenant

(i) un amino,
(ii) un radical de formule partielle (B), dans laquelle G est sélectionné parmi le groupe comprenant un alcanoyle inférieur non substitué ou substitué par un oxo, spécialement un acétoacétyle; un radical de formule partielle (C)
dans laquelle
$R_3$ est un radical de formule partielle (F)
dans laquelle
A' est -CH=CH-, de préférence en configuration trans, D' est un hydrogène, un sulfo ou de préférence un radical de formule partielle (A)
dans laquelle
$R_1$ est un hydrogène, un alkyle inférieur, spécialement un méthyle, un phényle ou un carboxy, de préférence un méthyle; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo; spécialement sélectionné parmi le groupe comprenant un hydrogène, un 5-chloro-2-hydroxyphénylazo, un 2-hydroxy-4-nitrophénylazo, un 2-hydroxy-5-sulfophénylazo, un 2-hydroxy-4-sulfo-1-naphtylazo et spécialement un 2-carboxyphénylazo; et chacun de m' et n' est 1, le groupement sulfo correspondant étant de préférence lié en position o par rapport au radical bivalent -A- dans la formule I;
et
$R_4$ est un halogéno, spécialement un chloro, ou un amino,
et
(iii) un radical de formule partielle (K)
dans laquelle
A''' est -CH=CH-, de préférence en configuration trans,
D''' est un hydrogène, un sulfo ou un radical de formule partielle (A) dans laquelle
$R_1$ est un hydrogène, un phényle, un carboxy ou de préférence un méthyle; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo; spécialement sélectionné parmi le groupe comprenant un hydrogène, un 5-chloro-2-hydroxyphénylazo, un 2-hydroxy-4-nitrophénylazo, un 2-hydroxy-5-sulfophénylazo, un 2-hydroxy-4-sulfo-1-naphtylazo et spécialement un 2-carboxyphénylazo; et chacun de m''' et n''' est 1, le groupement sulfo correspondant étant de préférence lié en position o par rapport au radical bivalent -A'''- dans la formule I,
ou un tautomère de celui-ci; ou un sel pharmaceutiquement acceptable ou un sel complexe pharmaceutiquement acceptable de celui-ci où au moins un groupement formant un sel ou formant un complexe est présent.

**3.** Composé suivant la revendication 1, pour une utilisation dans le traitement d'un animal, notamment l'homme, de formule I sélectionné parmi le groupe comprenant
l'acide trans-5-amino-2-[2-{4-(4,5-dihydro-3-méthyl-5-oxo-1H-pyrazol-1-yl)-2-sulfophényl}éthényl]benzènesulfonique;
la N,N'-bis(trans-4-{2-[4-(3-méthyl-5-oxo-4-(o-carboxyphénylazo)-4,5-dihydro-lH-pyrazol-1-yl)-2-sulfophényl]vinyl}-2-sulfophényl)urée; et
le complexe de cuivre de la N,N'-bis(trans-4-{2-[4-(3-méthyl-5-oxo-4-(o-carboxyphénylazo)-4,5-dihydro-1H-pyrazol-l-yl)-2-sulfophényl]vinyl}-2-sulfophényl)urée,
ou un sel pharmaceutiquement acceptable de celui-ci.

**4.** Composé suivant la revendication 1, pour une utilisation dans le traitement d'un animal, notamment l'homme, de formule I no mmé l'acide trans-5-(acétoacétylamino)-2-[2-{4-(4,5-dihydro-3-méthyl-5-oxo-1H-pyrazol-1-yl)-2-sulfophényl}éthényl]benzènesulfonique; ou un sel pharmaceutiquement acceptable de celui-ci.

**5.** Utilisation d'un composé de formule I, d'un tautomère de celui-ci, d'un sel ou d'un sel complexe de celui-ci suivant la revendication 1, pour la fabrication d'une préparation pharmaceutique pour l'inhibition de l'interaction d'un régulateur de transcription avec un élément de réponse rétroviral ou de fonctions spécifiques supplémentaires de Tat, comme une activation spécifique de processus cellulaires promouvant le VIH, spécialement l'expression de

corécepteurs cellulaires du VIH, spécialement CXCR4.

**6.** Utilisation d'un composé de formule I, d'un tautomère de celui-ci, d'un sel ou d'un sel complexe de celui-ci suivant la revendication 1, pour la fabrication d'une préparation pharmaceutique pour le traitement d'une maladie virale.

**7.** Utilisation d'un composé de formule I, d'un tautomère de celui-ci, d'un sel ou d'un sel complexe de celui-ci suivant la revendication 1, pour la fabrication d'une préparation pharmaceutique utile comme agent thérapeutique antirétrovirus inhibant l'interaction d'un régulateur de transcription avec un élément de réponse rétroviral ou inhibant des fonctions spécifiques supplémentaires de Tat, comme une activation de processus cellulaires promouvant le VIH, spécialement l'expression de corécepteurs cellulaires du VIH, spécialement CXCR4.

**8.** Composé de formule I,

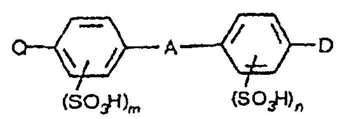

(I)

dans laquelle

A est $-CH_2-CH_2-$ ou -CH=CH-;
D est un radical de formule partielle (A),

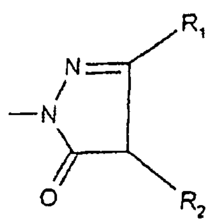

(A)

dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo, avec au moins un substituant hydroxy ou carboxy en position ortho par rapport au groupement azo, chacun de m et n est 1, 2, 3 ou 4; et
Q est un radical de formule partielle (C),

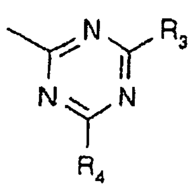

(C),

dans laquelle
$R_3$ est sélectionné parmi le groupe comprenant

(i) un radical de formule partielle (F),

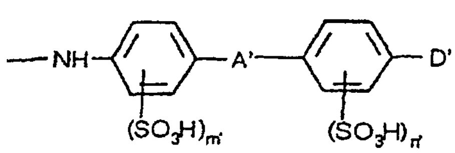

(F)

dans laquelle
A' est $-CH_2-CH_2-$ ou -CH=CH-,
D' est un hydrogène, un sulfo ou un radical de formule partielle (A)
dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo,
et chacun de m' et n' est 1, 2, 3 ou 4, de préférence 1,
(ii) un phénylamino dans lequel le phényle est substitué par un sulfophénylazo, par un (hydroxy et/ou carboxy)phénylazo, par un méthyle et un 3-méthyl-4-[(mono- ou disulfo)naphtylazo]phénylazo ou par un [2-(phénylazo)-2-(acétyl)acétyl]amino; et
(iii) un naphtylamino dans lequel le naphtyle est substitué par jusqu'à quatre radicaux sélectionnés parmi le groupe comprenant un hydroxy, un sulfo, un (phényl substitué par un hydroxy et/ou un chloro)azo, un (phényl substitué par un carboxy)azo et un (phényl substitué par un hydroxy et/ou un nitro)azo,
et
$R_4$ est un amino,

ou un tautomère de celui-ci; ou un sel ou un sel complexe de celui-ci où au moins un groupement formant un sel ou formant un complexe est présent.

**9.** Composé de formule I suivant la revendication 8,
dans laquelle

A est -CH=CH-, spécialement en configuration trans;
D est un radical de la formule partielle (A)
dans laquelle
$R_1$ est un alkyle inférieur, et
$R_2$ est un hydrogène,
chacun de m et n est 1, le groupement sulfo correspondant étant de préférence lié en position o par rapport au radical bivalent -A- dans la formule I; et
Q est un radical de formule partielle (C),
dans laquelle
$R_3$ est un radical de formule partielle (F),
dans laquelle
A' est -CH=CH-, spécialement en configuration trans,
D' est un radical de formule partielle (A)
dans laquelle
$R_1$ est un alkyle inférieur, spécialement un méthyle; et
$R_2$ est un hydrogène,
chacun de m et n est 1, le groupement sulfo correspondant étant de préférence lié en position o par rapport au radical bivalent -A- dans la formule I;
et
$R_4$ est un amino,
ou un tautomère de celui-ci; ou un sel ou un sel complexe de celui-ci où au moins un groupement formant un sel ou formant un complexe est présent.

**10.** Composé de formule I nommé
N,N'-bis(trans-4-{2-[4-(3-méthyl-5-oxo-4,5-dihydro-1H-pyrazol-1-yl)-2-sulfophényl]vinyl}-2-sulfophényl)-[1,3,5] triazine-2,4,6-triamine ou N,N' -(bis(trans-4-{2-[4-(3-méthyl-5-oxo-4,5-dihydro-lH-pyrazol-l-yl)-2-sulfophényl]vinyl}-2-sulfophényl)-2-chloro-[1,3,5]triazine-4,6-diamine;
ou un tautomère de celui-ci; ou sel pharmaceutiquement acceptable de celui-ci.

**11.** Composé de formule I, ou un tautomère de celui-ci; ou un sel ou un sel complexe de celui-ci où au moins un groupement formant un sel ou formant un complexe est présent suivant l'une quelconque des revendications 8 à 10, pour une utilisation dans le traitement d'un animal, notamment l'homme.

**12.** Préparation pharmaceutique comprenant un composé de formule I, ou un tautomère de celui-ci; ou un sel ou un

sel complexe de celui-ci où au moins un groupement formant un sel ou formant un complexe est présent suivant l'une quelconque des revendications 8 à 10 et un vecteur pharmaceutiquement acceptable.

**13.** Utilisation d'un composé de formule I, ou d'un tautomère de celui-ci; ou d'un sel ou d'un sel complexe de celui-ci où au moins un groupement formant un sel ou formant un complexe est présent suivant la revendication 1, pour la fabrication d'une préparation pharmaceutique utile comme agent thérapeutique antirétrovirus inhibant l'interaction d'un régulateur de transcription avec un élément de réponse rétroviral ou inhibant des fonctions spécifiques supplémentaires de Tat, comme une activation de processus cellulaires promouvant le VIH, spécialement l'expression de corécepteurs cellulaires du VIH, spécialement CXCR4.

**14.** Procédé pour la préparation d'un composé de formule I, ou d'un tautomère de celui-ci; ou d'un sel ou d'un sel complexe de celui-ci où au moins un groupement formant un sel ou formant un complexe est présent suivant la revendication 8, dans lequel on fait réagir avec de l'ammoniac un composé de formule II,

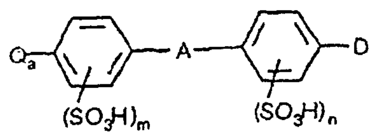

(II)

dans laquelle

$Q_a$ est un radical de formule partielle (C),

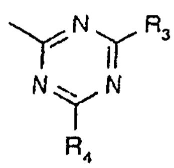

(C),

dans laquelle
$R_3$ est sélectionné parmi le groupe comprenant

(i) un radical de formule partielle (F),
dans laquelle
A' est $-CH_2-CH_2-$ ou -CH=CH-,
D' est un hydrogène, un sulfo ou un radical de formule partielle (A)
dans laquelle
$R_1$ est un hydrogène, un alkyle inférieur, un phényle ou un carboxy; et
$R_2$ est un hydrogène, un phénylazo ou un naphtylazo dans lequel le phényle ou le naphtyle est non substitué ou substitué par un ou plusieurs substituants sélectionnés parmi le groupe comprenant un halogéno, un hydroxy, un carboxy, un nitro et un sulfo,
et chacun de m' et n' est 1, 2, 3 ou 4,
(ii) un phénylamino dans lequel le phényle est substitué par un sulfophénylazo, par un (hydroxy et/ou carboxy)phénylazo, par un méthyle et un 3-méthyl-4-[(mono- ou disulfo)naphtylazo]phénylazo ou par un [2-(phénylazo)-2-(acétyl)acétyl]amino; et
(iii) un naphtylamino dans lequel le naphtyle est substitué par jusqu'à quatre radicaux sélectionnés parmi le groupe comprenant un hydroxy, un sulfo, un (phényl substitué par un hydroxy et/ou un chloro)azo, un (phényl substitué par un carboxy)azo et un (phényl substitué par un hydroxy et/ou un nitro)azo,
et
$R_4$ est un halogéno, et

A, D, m, et n ont les significations données pour un composé de formule I à la revendication 7,
et, si on le souhaite, on convertit un composé libre de formule I ou un tautomère de celui-ci pouvant être obtenu suivant ce procédé en un sel ou un sel complexe, on convertit un sel ou un sel complexe d'un composé de formule I ou d'un tautomère de celui-ci pouvant être obtenu, en un sel ou sel complexe différent ou en un composé libre de formule I ou en un tautomère de celui-ci, et/ou un mélange d'isomères d'un composé obtenu

de formule I est séparé en ses isomères individuels.